# EUROPEAN PATENT APPLICATION

(11) **EP 4 169 921 A1**
(43) Date of publication of application: **26.04.2023**
(21) Application number: 21831769.1
(22) Date of filing: 24.06.2021
(51) Int. Cl.: C07D 471/04, A61K 31/437, A61P 29/00, A61P 11/14, A61P 13/00, A61P 11/00, A61P 25/04

(54) **CRYSTALLINE FORM OF HETEROCYCLIC COMPOUND, PREPARATION METHOD THEREFOR AND APPLICATION THEREOF**

(30) Priority: 29.06.2020 CN 202010610052
(71) Applicant: Wuhan LL Science and Technology Development Co., Ltd., Wuhan, Hubei 430075 (CN)
(72) Inventor: WANG, Liang, Wuhan, Hubei 430075 (CN); LOU, Jun, Wuhan, Hubei 430075 (CN); HONG, Huayun, Wuhan, Hubei 430075 (CN); GUO, Xiaodan, Wuhan, Hubei 430075 (CN); QIAN, Lina, Wuhan, Hubei 430075 (CN); ZHANG, Yihan, Wuhan, Hubei 430075 (CN); CHEN, Yongkai, Wuhan, Hubei 430075 (CN); WANG, Chaodong, Wuhan, Hubei 430075 (CN)
(74) Representative: Prüfer & Partner mbB Patentanwälte · Rechtsanwälte
(86) International application number: PCT/CN2021/102068
(87) International publication number: WO 2022/001820

(57) **Abstract**

Provided are a crystalline form of a compound represented by formula A, the crystalline form being crystalline form I, crystalline form II, crystalline form III, crystalline form IV, crystalline form V, crystalline form VI, crystalline form VII, crystalline form VIII or crystalline form IX, a preparation method therefor, a composition thereof, and an application thereof in the preparation of a P2X3 receptor antagonist or an application thereof in the preparation of drugs preventing and/or treating pains, urinary tract illness or respiratory system illness. The compound has high P2X3 antagonist activity, and has good selectivity, low toxicity, good metabolic stability and little taste influence.

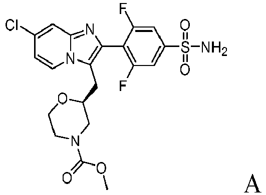

## Description

This application claims the right of the following priority of Chinese patent application 2020106100526 filed on June 29, 2020. The contents of the above Chinese patent application are incorporated herein by reference in its entireties.

### TECHNICAL FIELD

The present disclosure relates to the field of medicinal chemistry, and in particular relates to a crystalline form of heterocyclic compound, a preparation method therefor and a use thereof.

### BACKGROUND

ATP receptors are classified into two main families, P2Y- and P2X-purinoceptors, based on their molecular structure, transduction mechanism, and pharmacological properties. P2X-purinoceptor is a family of ATP-gated cation channels, and several subtypes have been cloned, including: six homopolymer receptors, P2X1, P2X2, P2X3, P2X4, P2X5, and P2X7; and three homopolymer receptors P2X2/3, P2X4/6, P2X1/5. It has been found that P2X3 receptor is specifically expressed in the primary afferent nerve fibers of "hollow viscera", such as lower urinary tract and respiratory tract.

Cough is the main symptom of respiratory diseases, and 70 % to 80 % of patients in respiratory clinics have cough symptoms. With the increasing prevalence of COPD and IPF and the like, cough is the main symptom of most respiratory diseases, and the demand is also increasing. As the body's defensive nerve reflex, cough is beneficial to clearing respiratory secretions and harmful factors, but frequent and severe cough will seriously affect patients' work, life and social activities.

There are not many varieties of P2X3 antagonists specifically developed for cough indications, and the project with rapid progress is Roche's AF-219 project, which has a good effect on refractory cough in the latest completed phase II clinical trial, but taste disturbance is a serious problem.

At present, there is no drug marketed for inhibiting P2X3 pathway to treat many diseases including chronic cough. Therefore, the development of novel compounds that can inhibit the activity of P2X3 has positive significance for the treatment of diseases.

The patent application CN201911379293.8 involves a P2X3 antagonist as shown below, which has high P2X3 antagonistic activity, and has good selectivity, low toxicity, good metabolic stability, and little taste influence. And the antagonist has good prospects for drug development. However, it does not involve the crystalline form of the compound.

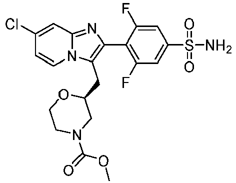

Compound generally have polymorphism, and general drug may exist in two or more different crystalline forms. The existing form and quantity of polymorphic compounds are unpredictable, and different crystalline forms of the same drug have significant differences in solubility, melting point, density, stability, etc., which affect the temperature type, homogeneity, bioavailability, efficacy and safety of the drug to varying degrees. Therefore, in the process of new drug research and development, it is necessary to conduct a comprehensive polymorphic screening of compounds, and it is of great clinical significance to select the crystalline form suitable for the development of pharmaceutical preparations.

### CONTENT OF THE PRESENT INVENTION

The present disclosure provides a crystalline form of heterocyclic compound and a preparation method therefor and a use thereof. The crystalline forms of the present disclosure have a good stability and have an important value in terms of the optimization and development of drugs.

The present disclosure provides a crystalline form of the compound of formula A or a solvate thereof: 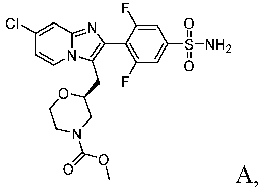 the crystalline form is selected from crystalline form I, crystalline form II, crystalline form III, crystalline form IV, crystalline form V, crystalline form VI, crystalline form VII crystalline form VIII and crystalline form IX.

The present disclosure provides a crystalline form III of the compound of formula A, wherein, the crystalline form III has an X-ray powder diffraction pattern (XRPD) comprising characteristic diffraction peaks at the following angles of 2θ: 12.91°± 0.20°, 16.77° ± 0.20°, 19.27° ± 0.20° and 22.80° ± 0.20°.

In some preferred embodiments of the present disclosure, the XRPD of the crystalline form III also comprises characteristic peaks at the following angle 2θ: 13.75°±0.20°, 14.46°±0.20° and 20.86°±0.20°; further comprises characteristic peaks at the following angle 2θ: 21.08°±0.20°, 23.75°±0.20° and 24.05°±0.20°.

In some preferred embodiments of the present disclosure, the crystalline form III has an XRPD pattern represented by 2θ angle substantially as shown in Fig.1. In thermogravimetric analysis (TGA) pattern of the crystalline form III, the crystalline form III has a weight loss of 1.5% in the range from room temperature (RT) to 100°C, and the "%" is the weight percentage. In differential scanning calorimetric (DSC) pattern of the crystalline form III, the first endothermic peak is the removal of 0.4 water, and the second endothermic peak is attributed to the melting endothermic peak after sample dehydration, and the TGA and DSC pattern are preferably as shown in Fig.2. After the crystalline form III is dehydrated, the crystalline quickly absorbs moisture under ambient humidity and becomes crystalline form III again, and the XRPD pattern before and after heating to dehydration are preferably as shown in Fig.3. The dynamic moisture adsorption (DVS) pattern of the crystalline form III shows that the sample has certain hygroscopicity, and the water content changes little in a wide humidity range, and the DVS pattern is preferably as shown in Fig.4. In the XPRD pattern before and after the DVS test of the crystalline form III, there is no obvious change in the XRPD before and after the DVS test, and the XPRD pattern before and after the DVS test are preferably as shown in Fig.5. In polarized light microscope (PLM) pattern of the crystalline form III, the crystalline form is an irregular crystal of about 2 µm, and the agglomeration is 20 to 50 µm, and the PLM pattern is preferably substantially as shown in Fig.6. The purity of the crystalline form III is generally above 90%, preferably above 95%.

The present disclosure provides a crystalline form V of the compound of formula A, wherein, the crystalline form V has an XRPD comprising characteristic diffraction peaks at the following angles of 2θ: 8.38 °± 0.20°, 9.15° ± 0.20°, 13.52° ± 0.20° and 18.44 ± 0.20°.

In some preferred embodiments of the present disclosure, the XRPD of the crystalline form V also comprises characteristic peaks at the following angle 2θ: 16.26°±0.20°, 16.89°±0.20° and 17.86°±0.20°; further comprises characteristic peaks at the following angle 2θ: 22.35°±0.20°, 23.56°±0.20° and 24.74°±0.20°.

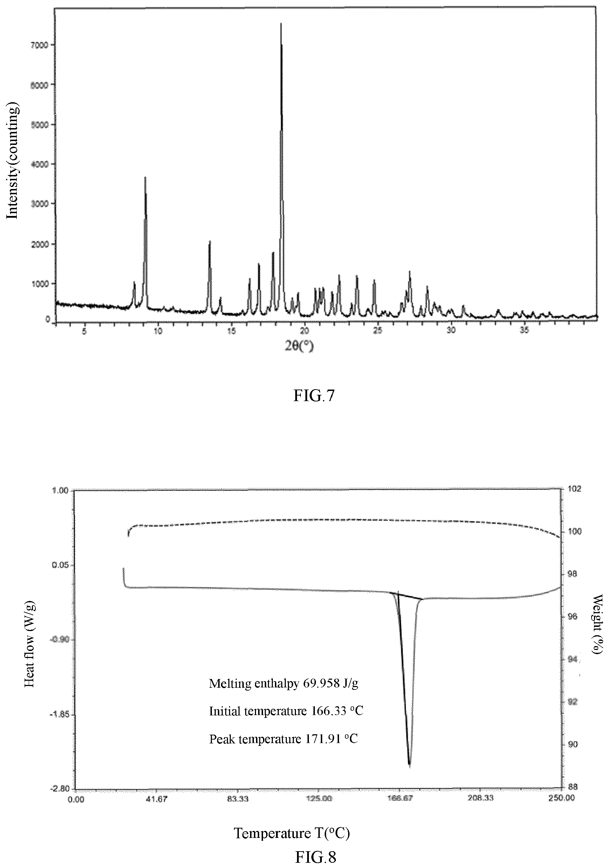

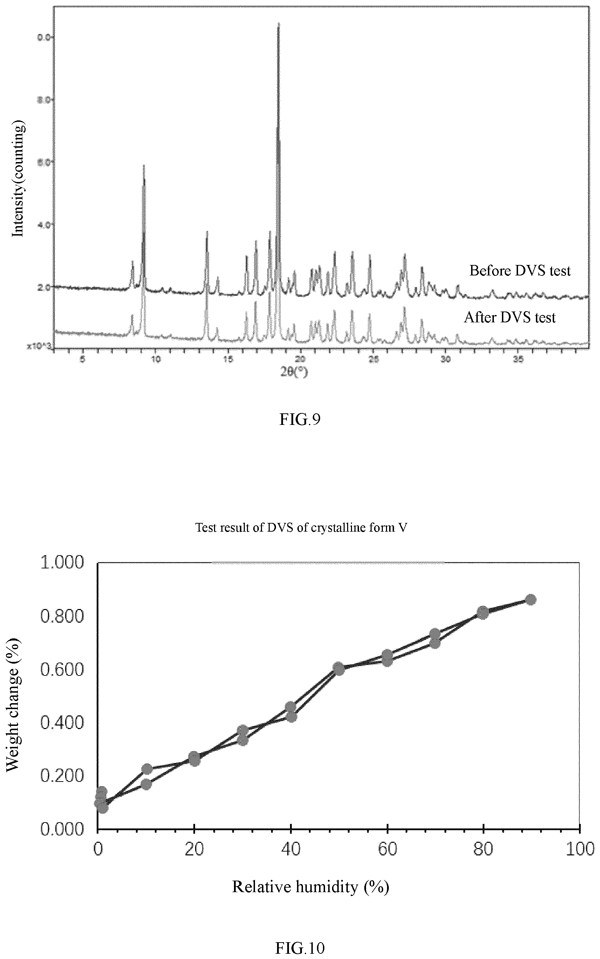

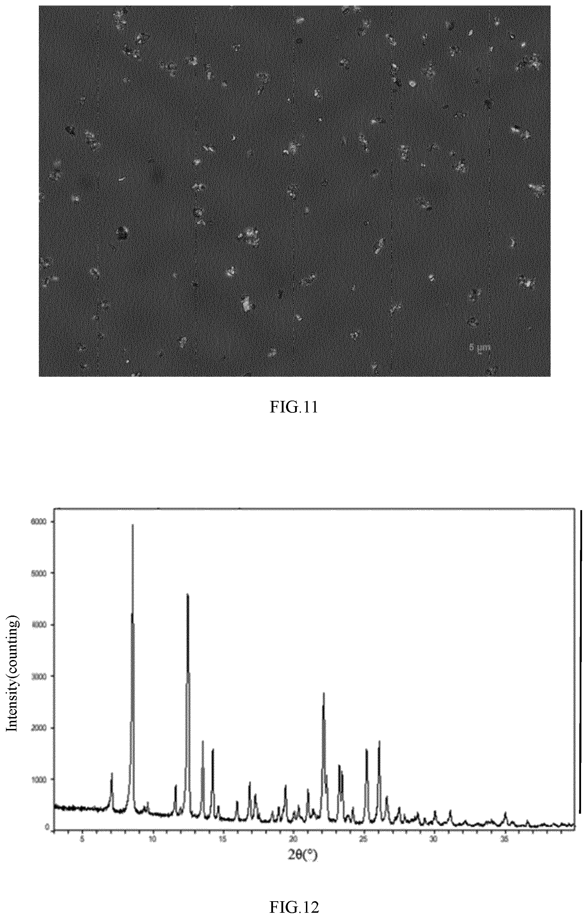

In some preferred embodiments of the present disclosure, the crystalline form V has an XRPD pattern represented by 2θ angle substantially as shown in Fig.7. In TGA pattern of the crystalline form V, there is no weight loss in the temperature range of RT to 230°C. In DSC pattern of the crystalline form V, there is an endothermic peak at 166 °C ± 2 °C, and the melting enthalpy is 70±2J/g, and the TGA and DSC pattern is preferably as shown in Fig.8. Combined with DSC and TGA pattern, it can be seen that the product is an anhydrous crystalline form. The DVS pattern of the crystalline form V shows that the sample has certain hygroscopicity (0.7%, 80%RH), and the DVS pattern is preferably as shown in Fig.9. In the XPRD pattern before and after the DVS test of the crystalline form V, there is no obvious change in the XRPD before and after the DVS test, and the XPRD pattern before and after the DVS test is preferably as shown in Fig.10. In PLM pattern of the crystalline form V, the crystalline form is an irregular crystal of about 5 µm, and the PLM pattern is preferably substantially as shown in Fig.11. The purity of the crystalline form V is generally above 90%, preferably above 95%.

The present disclosure provides a crystalline form I of the compound of formula A, wherein, the crystalline form I has an XRPD comprising characteristic diffraction peaks at the following angles of 2θ: 8.56° ± 0.20°, 12.48° ± 0.20° and 22.13° ± 0.20°.

In some preferred embodiments of the present disclosure, the X-ray powder diffraction pattern of the crystalline form I also comprises characteristic peaks at the following angle 2θ: 13.53°±0.20°, 14.25°±0.20°, 25.18°±0.20° and 26.07°±0.20°; further comprises characteristic peaks at the following angle 2θ: 22.32°±0.20°, 23.23°±0.20° and 23.42°±0.20°.

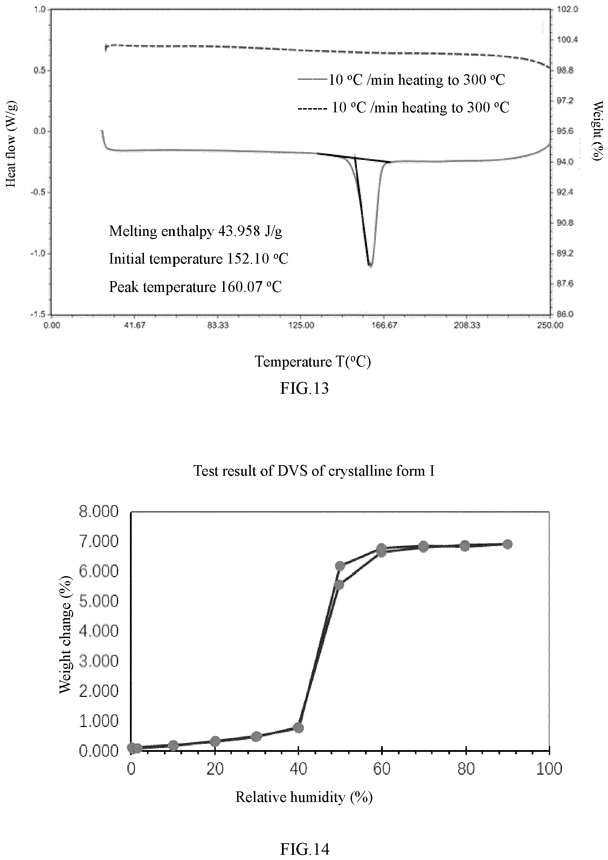

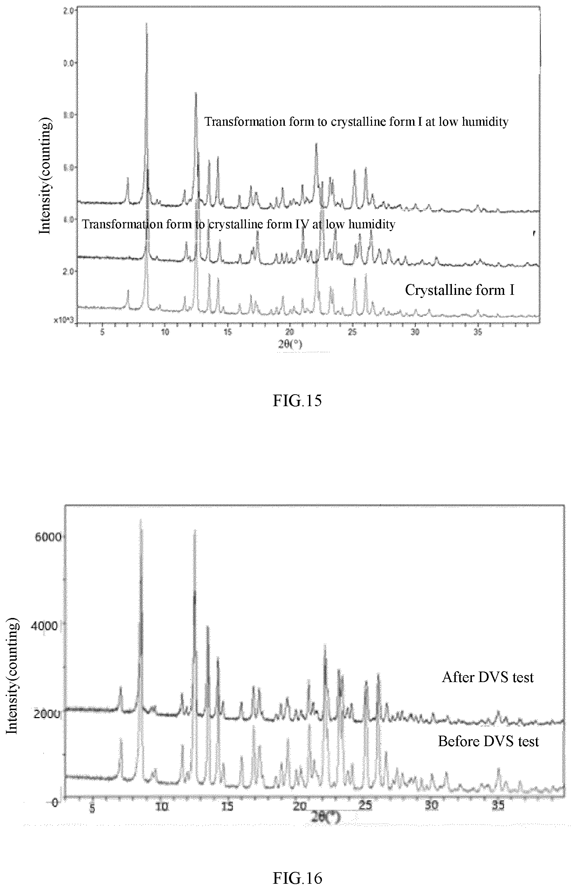

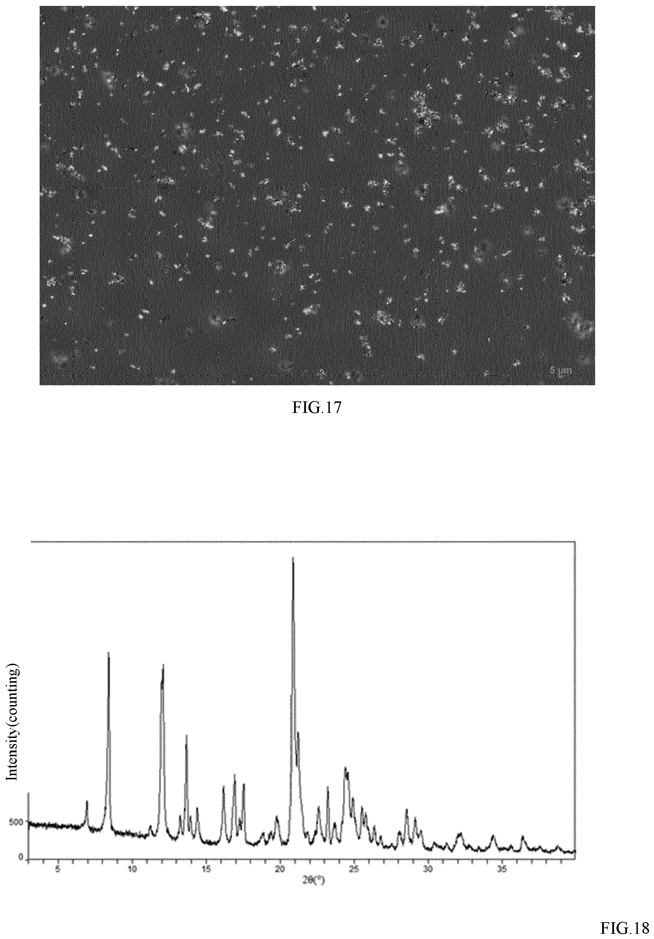

In some preferred embodiments of the present disclosure, the crystalline form I has an XRPD pattern represented by 2θ angle substantially as shown in Fig.12. In DSC pattern of the crystalline form I, there is an endothermic peak at 152 °C ± 2 °C, and the melting enthalpy is 44±2J/g. In TGA pattern of the crystalline form I, there is no weight loss in the temperature range of RT to 230°C. TGA and the DSC pattern is as shown in Fig. 13. Combined with DSC and TGA pattern, it can be seen that the product is an anhydrous crystalline form. The DVS pattern of the crystalline form I is as shown in Fig. 14, and when the relative humidity is greater than 40%, the weight of the crystalline form I increases sharply. When the relative humidity drops to 40%, all the absorbed water is discharged. In the XRPD pattern of the crystalline form I after moisture absorption, the crystalline form I absorbs moisture and is transformed into a hydrate of crystalline form IV under high humidity environment, which is then dried in a vacuum oven at 30°C and then transformed into the initial crystalline form I, and the XRPD pattern before and after moisture absorption are preferably substantially as shown in Fig. 15. It can be seen that combined with the DVS test results (Fig. 14), when the relative humidity of the environment is higher than 40%, crystalline form I rapidly absorbs moisture and is transformed into hydrate; and when the relative humidity is less than 50%, the adsorbed water is rapidly removed and transformed into crystalline form I. That is, the transformation between crystalline forms I and IV is reversible. The crystalline form I shows hygroscopicity (6.8%, 80%RH), and the crystalline form of the crystalline form I remains unchanged after the DVS test, and the XRPD pattern before and after the DVS test are as shown in Fig. 16. In the PLM pattern of the crystalline form I, the crystalline form is an irregular crystal of about 5 µm, and the PLM pattern is preferably substantially as shown in Fig. 17. The purity of the crystalline form I is generally above 90%, preferably above 95%.

The present disclosure provides a crystalline form II of a MTBE solvate of the compound of formula A, wherein, the crystalline form II has an XRPD comprising characteristic diffraction peaks at the following angles of 2θ: 8.42 °± 0.20°, 12.09° ± 0.20°, 13.68° ± 0.20° and 20.87° ± 0.20°.

In some preferred embodiments of the present disclosure, the XRPD of the crystalline form II also comprises characteristic peaks at the following angle 2θ: 16.17°±0.20°, 16.93°±0.20°, 17.55°±0.20° and 21.20°±0.20°; further comprises characteristic peaks at the following angle 2θ: 22.60°±0.20°, 23.23°±0.20° and 24.40°±0.20°.

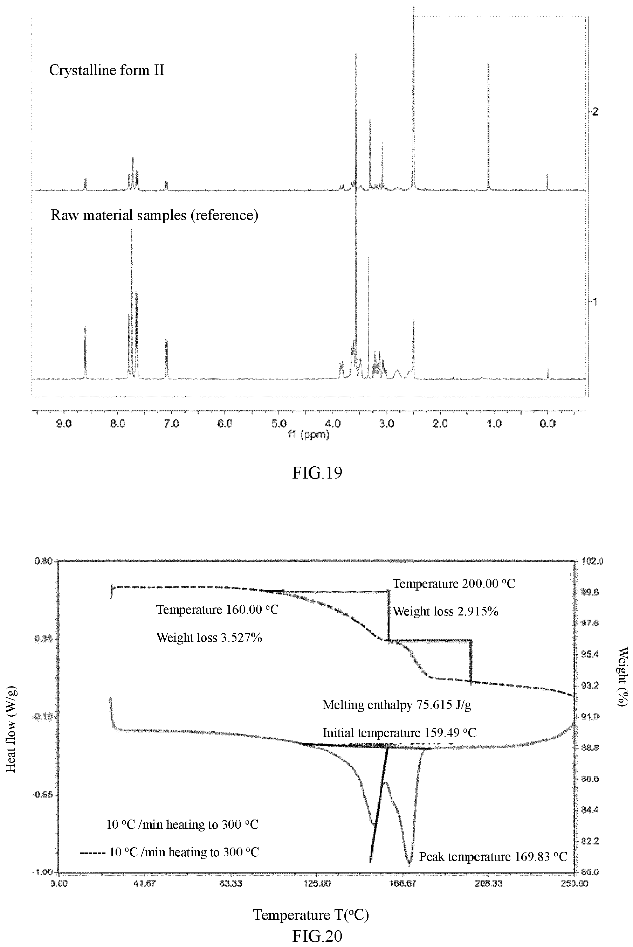

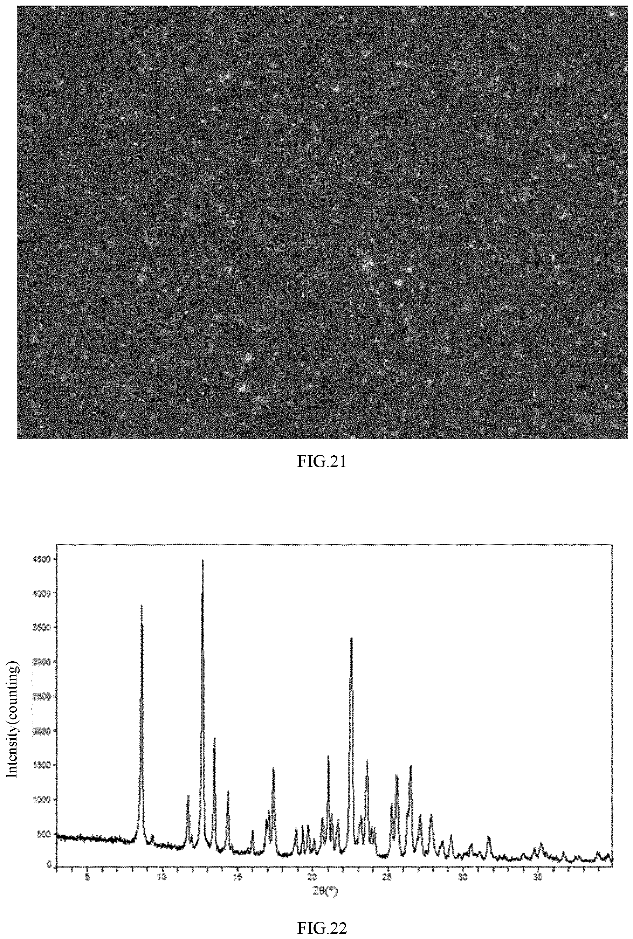

In some preferred embodiments of the present disclosure, the crystalline form II has an XRPD pattern represented by 2θ angle substantially as shown in Fig.18. In the nuclear magnetic resonance (HNMR) pattern of crystalline form II, there are residual signals of MTBE at the chemical shifts of 1.11 and 3.08, and the HNMR pattern of residual MTBE is as shown in Fig. 19. In TGA pattern of crystalline form II, there is a weight loss of 3.5% in the temperature range of 100 to 160 °C and a weight loss of 2.9% in the range of 160 to 200 °C. In DSC pattern of the crystalline form II, there are two adjacent endothermic peaks, and the TGA and DSC pattern are preferably as shown in Fig.20. Compared with HNMR pattern of the residual MTBE as shown in Fig.19, it can be seen that the product is a MTBE solvate. In the PLM pattern of the crystalline form III, the crystalline form is an irregular crystal of about 2 µm, and the PLM is preferably substantially as shown in Fig.21.

The present disclosure provides a crystalline form IV of a hydrate of the compound of formula A, wherein, the crystalline form IV has an XRPD comprising characteristic diffraction peaks at the following angles of 2θ: 8.65° ± 0.20°, 12.69° ± 0.20° and 22.56° ± 0.20°.

In some preferred embodiments of the present disclosure, the XRPD of the crystalline form IV also comprises characteristic peaks at the following angle 2θ: 13.48°±0.20°, 17 39°±0.20°, 21.04°±0.20° and 23.63°±0.20°; further comprises characteristic peaks at the following angle 2θ: 14.39°±0.20°, 25.60°±0.20° and 26.52°±0.20°.

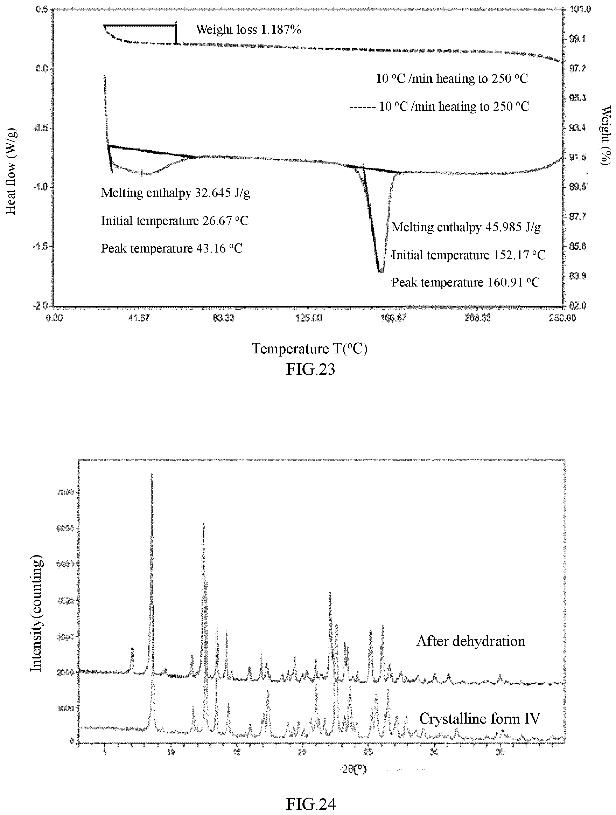

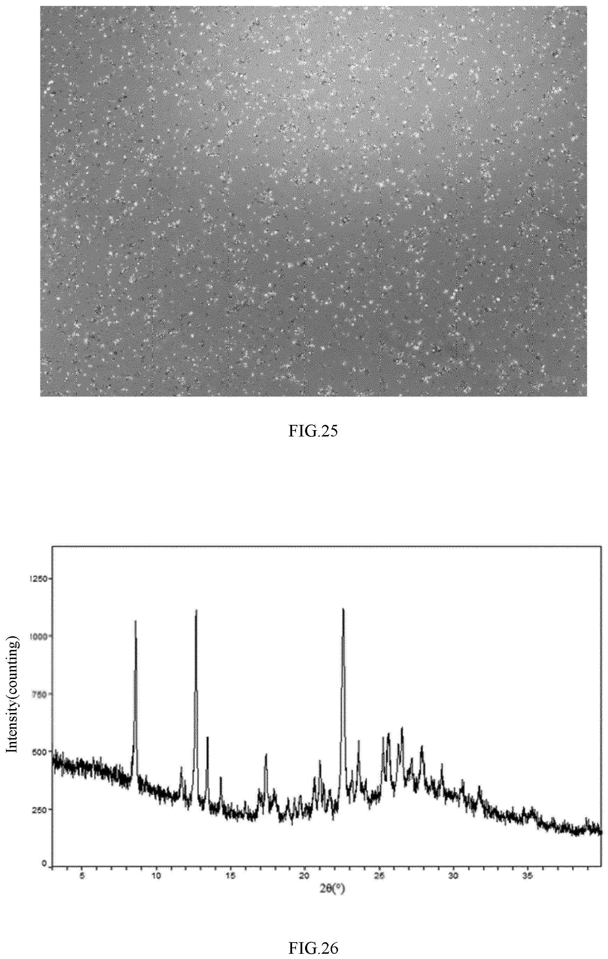

In some preferred embodiments of the present disclosure, the crystalline form IV has an XRPD pattern represented by 2θ angle substantially as shown in Fig.22. In TGA pattern of the crystalline form IV, there is a weight loss of 1.2% in the range of RT to 60°C. In DSC pattern of the crystalline form IV, there are two adjacent endothermic peaks, the first broad endothermic peak is presumed to be caused by dehydration, and the subsequent endothermic peak is a melting peak, and the TGA and DSC pattern are preferably as shown in Fig.23. The crystalline form IV is stable only in a high humidity environment, and after dehydration, the crystalline form IV quickly absorbs moisture under ambient humidity and becomes crystalline form I again, and the XRPD pattern before and after heating to dehydration are preferably as shown in Fig.24. In the PLM pattern of the crystalline form IV, the crystalline form is an irregular crystalline of about 5 µm, and the PLM pattern is preferably substantially as shown in Fig.25. The purity of the crystalline form IV is generally above 90%, preferably above 95%.

The present disclosure provides a crystalline form VI of a hydrate of the compound of formula A, wherein, the crystalline form VI has an X-ray powder diffraction pattern comprising characteristic diffraction peaks at the following angles of 2θ: 8.62° ± 0.20°, 12.69° ± 0.20° and 22.59° ± 0.20°.

In some preferred embodiments of the present disclosure, the XRPD of the crystalline form VI also comprises characteristic peaks at the following angle 2θ: 13.46°± 0.20°, 17.41°±0.20°, 26.51°±0.20°, 25.62°±0.02° and 25.24°±0.20°; further comprises characteristic peaks at the following angle 2θ: 23.64°±0.20°, 21.00°±0.20° and 27.85°±0.20°.

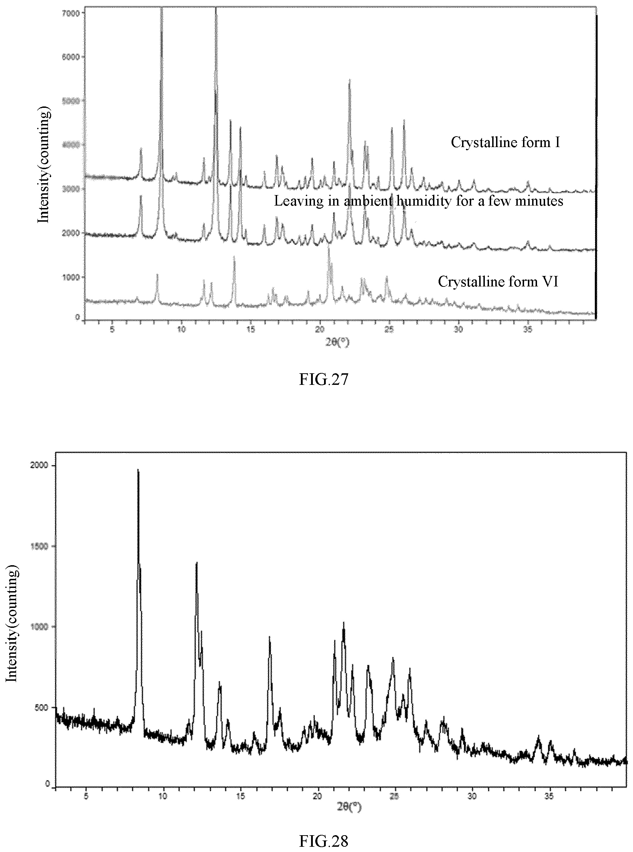

In some preferred embodiments of the present disclosure, the crystalline form VI has a XRPD pattern represented by 2θ angle substantially as shown in Fig.26. In the XRPD superimposition pattern of the crystalline form VI, the crystalline form sample is transformed into crystalline form I after being placed under ambient humidity (35% RH) for a few minutes. The XRPD pattern is as shown in Fig.27. This indicates that the crystalline form VI may be an extremely unstable hydrate. The purity of the crystalline form VI is generally above 90%, preferably above 95%.

The present disclosure provides a crystalline form VII of an ethylene glycol solvate of the compound of formula A, wherein, the crystalline form VII has an X-ray powder diffraction pattern comprising characteristic diffraction peaks at the following angles of 2θ: 8.36°± 0.20°, 12.13 °± 0.20°, 12.45° ± 0.20°, 16.84° ± 0.20° and 21.66° ± 0.20°.

In some preferred embodiments of the present disclosure, the XRPD of the crystalline form VII also comprises characteristic peaks at the following angle 2θ: 21.07°±0.20° and 24.82°±0.20°; further comprises characteristic peaks at the following angle 2θ: 13.61°±0.20°, 23.22°±0.20° and 24.57°±0.20°.

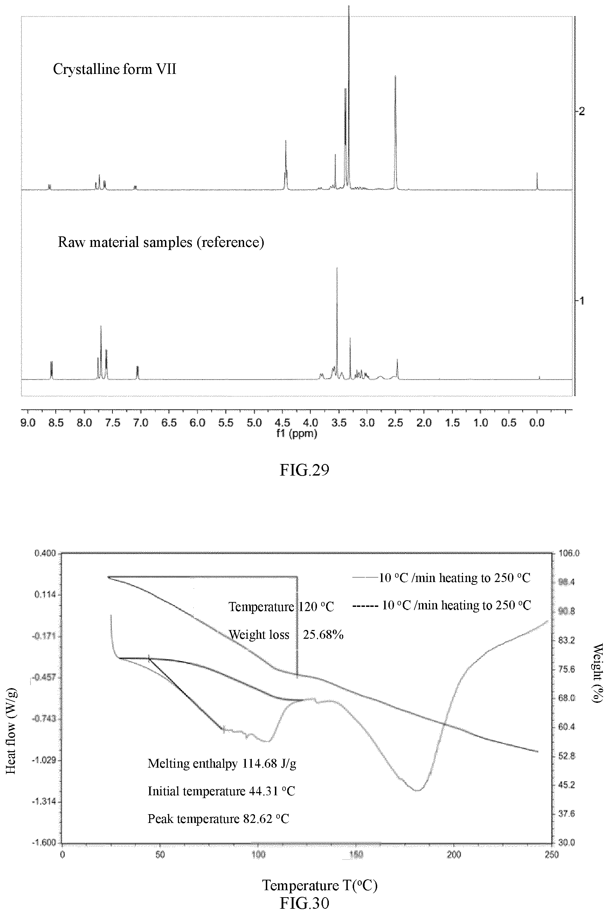

In some preferred embodiments of the present disclosure, the crystalline form VII has an XRPD pattern represented by 2θ angle substantially as shown in Fig.28. In ¹HNMR pattern of crystalline form VII, the chemical shifts δ at 3.39 and 4.44 show residual ethylene glycol solvent, and the ¹HNMR pattern of the residual ethylene glycol is preferably as shown in Fig.29. In TGA pattern of the crystalline form VII, there is a weight loss of 25.7% in the range of RT to 25.7°C. In DSC pattern of the crystalline form VII, there are two broad endothermic peaks, the first endothermic peak is presumed to be caused by desolventization, and the TGA and DSC pattern are preferably as shown in Fig.30. Combined with DSC and TGA pattern, it can be seen that the product is an ethylene glycol solvate. The purity of the crystalline form VII is generally above 90%, preferably above 95%.

The present disclosure provides a crystalline form VIII of a THF solvate of the compound of formula A, wherein, the crystalline form VII has an X-ray powder diffraction pattern comprising characteristic diffraction peaks at the following angles of 2θ: 8.53°±0.20°, 12.38° ± 0.20°, 13.66° ± 0.20° and 21.49° ± 0.20°.

In some preferred embodiments of the present disclosure, the X-ray powder diffraction pattern of the crystalline form VIII also comprises characteristic peaks at the following angle 2θ: 20.99°±0.20°, 24.94°±0.20° and 25.31°±0.20°; further comprises characteristic peaks at the following angle 2θ: 17.14°±0.20°, 21.72°±0.20° and 23.00°±0.20°.

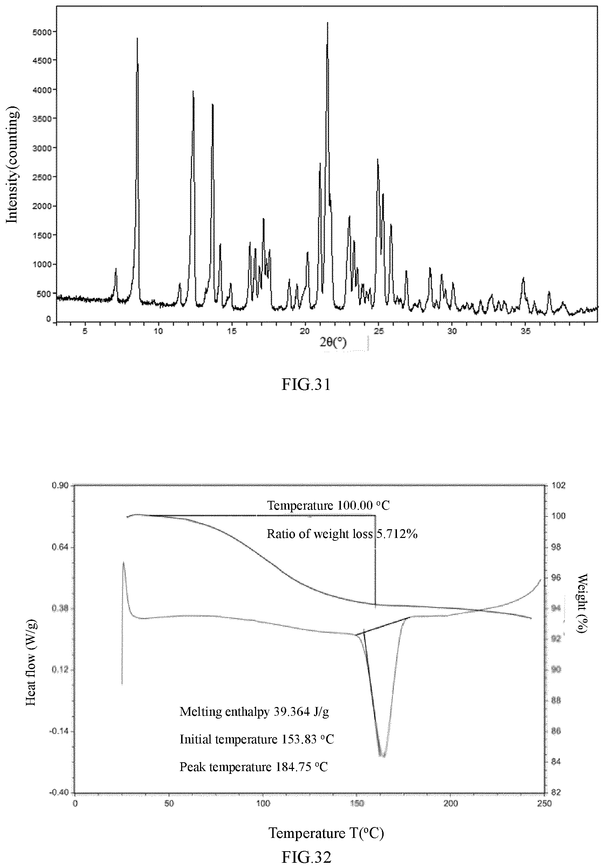

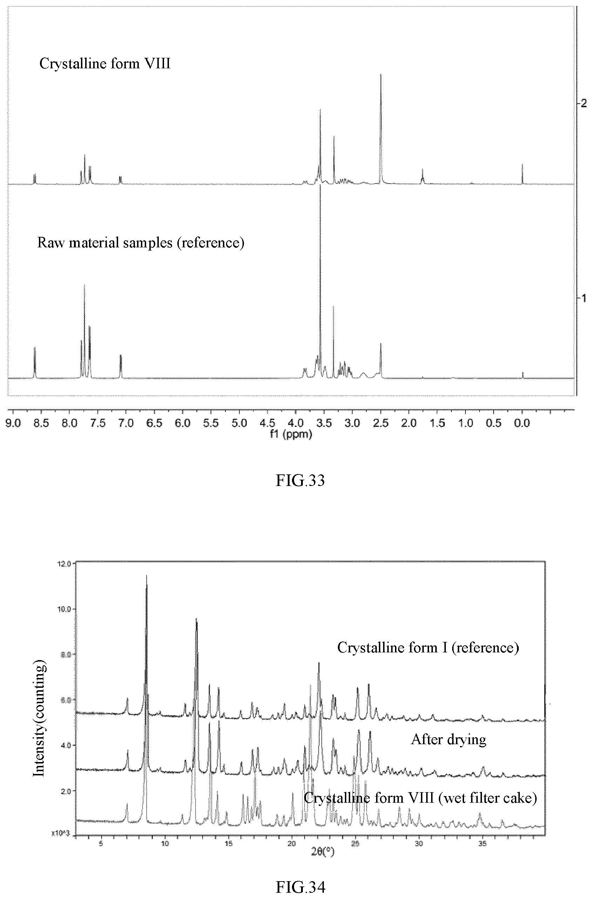

In some preferred embodiments of the present disclosure, the crystalline form VIII has an XRPD pattern represented by 2θ angle substantially as shown in Fig.31. In TGA pattern of the crystalline form VIII, there is a weight loss of 5.7% in the range of RT to 60°C. In the DSC pattern of the crystalline form VIII, there is only one endothermic peak, which is the melting peak of the sample after desolventization. Therefore, the crystalline form VIII is a solvate containing THF, and the TGA and DSC pattern are preferably as shown in Fig.32. In the ¹HNMR pattern of crystalline form VIII, the chemical shifts δ at 1.76 and 3.60 show residual THF solvent, and the ¹HNMR pattern is preferably as shown in Fig.33. The crystalline form VIII is unstable, and after desolventization (vacuum drying at 40°C for 3 hours), it is transformed into crystalline form I, and the XRPD pattern before and after drying are preferably as shown in Fig.34. The purity of the crystalline form VIII is generally above 90%, preferably above 95%.

The present disclosure provides a crystalline form IX of a DMSO solvate of the compound of formula A, wherein, the crystalline form IX has an XRPD comprising characteristic diffraction peaks at the following angles of 2θ: 8.55 °± 0.20°, 12.43° ± 0.20°, 21.75° ± 0.20° and 25.07° ± 0.20°.

In some preferred embodiments of the present disclosure, the XRPD of the crystalline form IX also comprises characteristic peaks at the following angle 2θ: 13.57°±0.20°, 17.18°±0.20°, 20.94°±0.20° and 25.57°±0.20°; further comprises characteristic peaks at the following angle 2θ: 21.37°±0.20° and 23.12°±0.20°.

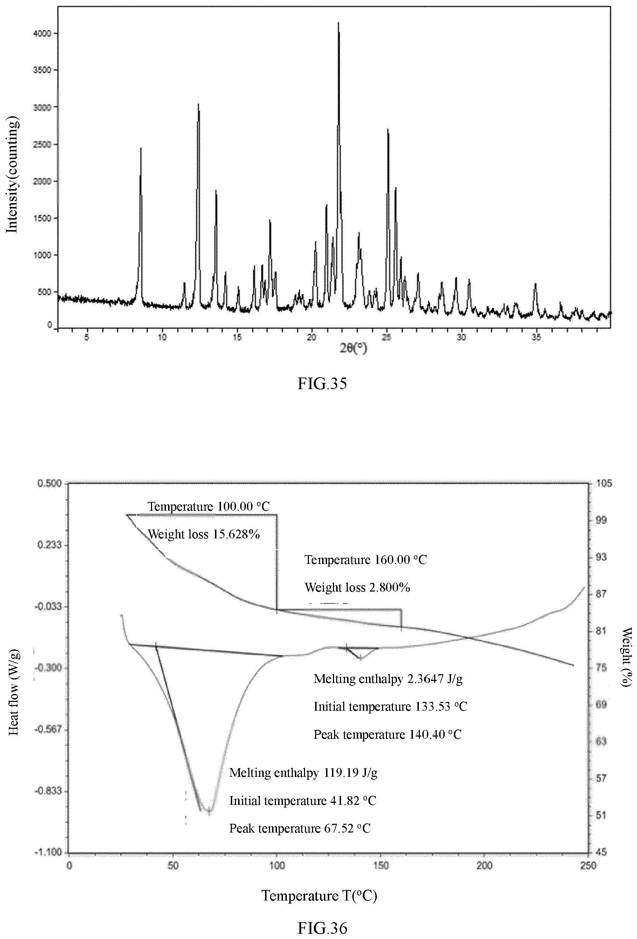

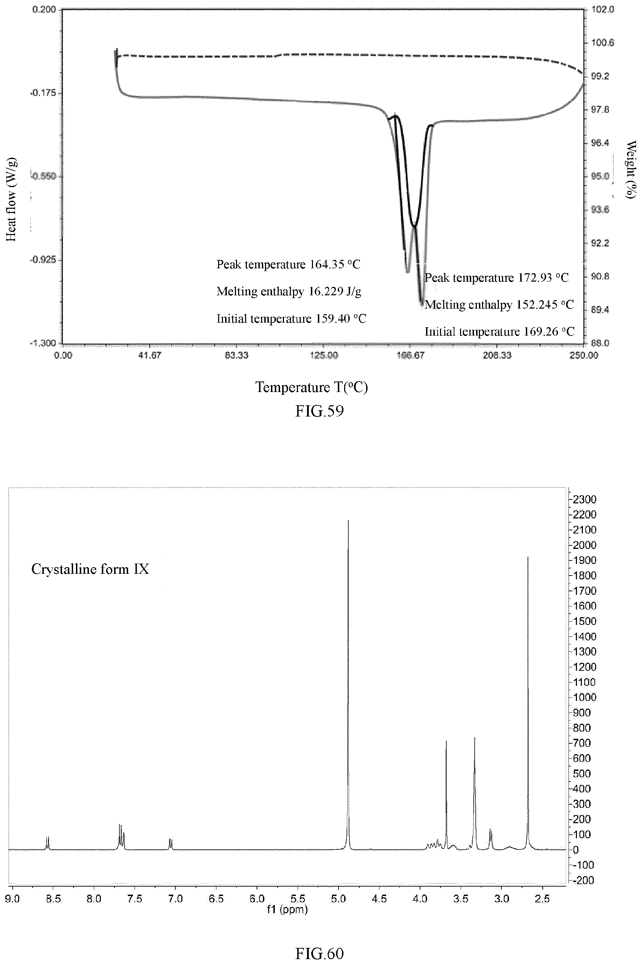

In some preferred embodiments of the present disclosure, the crystalline form IX has an XRPD pattern represented by 2θ angle substantially as shown in Fig.35. In TGA pattern of the crystalline form IX, the weight loss in the temperature range of RT to 160°C is 18.23%, and there is a corresponding endothermic peak corresponding to the TGA weight loss on the DSC pattern, and the TGA and DSC pattern are preferably as shown in Fig.36. In the ¹HNMR pattern of crystalline form IX, the chemical shift δ at 2.68 shows residual DMSO solvent, and the ¹HNMR pattern is preferably as shown in Fig.60. Combined with DSC and TGA pattern, it can be seen that the product is a DMSO solvate. The purity of the crystalline form IX is generally above 90%, preferably above 95%.

In the present disclosure, the XRPD pattern is measured using the spectra with Kα spectra.

In the present disclosure, the X-ray powder diffraction pattern is measured using the target type with Cu target.

The present disclosure also provides a preparation method of a crystalline form III of substance A, wherein, the preparation method is scheme 1, scheme 2 or scheme 3;
scheme 1 comprises the following steps: transforming the suspension of the amorphous form of the compound of formula A and a solvent to a crystalline form III of substance A; the solvent is water or alcohol solvent;
scheme 2 comprises the following steps: adding anti-solvent into a solution of the compound of formula A and a solvent, and crystallizing into a crystalline form III of substance A; the solvent is selected from the group consisting of alcohols, furans and DMSO; and the anti-solvent is water;
scheme 3 comprises the following steps: adding a solution of compound A and a solvent into aqueous solution A, and crystallizing into a crystalline form III of substance A, the aqueous solution A is the suspension of crystal seeds of crystalline form III of substance A and water; and the solvent is DMSO.

In the preparation method of the crystalline form III, when the scheme 1 is adopted, the solvent is preferably water or methanol.

In the preparation method of the crystalline form III, when the scheme 1 is adopted, the temperature of crystallization is 20 to 50°C, preferably 40°C or 50°C.

In the preparation method of the crystalline form III, when the scheme 1 is adopted, the mass volume ratio of the amorphous form of the compound of formula A to the solvent is 50 mg/mL.

In the preparation method of the crystalline form III, when the scheme 2 is adopted, the solvent is preferably selected from the group consisting of methanol, tetrahydrofuran and DMSO.

In the preparation method of the crystalline form III, when the scheme 2 is adopted, the volume ratio of the solvent to water is 3:1 to 1:1 (for example, 1:1 or 3:1).

In the preparation method of the crystalline form III, when the scheme 3 is adopted, the volume ratio of the solvent to water is 1:1 to 1:4 (for example, 1:1, 2:3, 1:2 or 1:4).

In the preparation method of the crystalline form III, when the scheme 1 is adopted, the preparation method preferably comprises the following steps: transforming the suspension of the amorphous form of compound A and the solvent; and the solvent is water or methanol. The temperature of stirring is 20 to 50°C, preferably 40°C. The mass volume ratio of the amorphous form of compound A to the solvent is 50 mg/mL.

In the preparation method of the crystalline form III, when the scheme 2 is adopted, the preparation method of the crystalline form III preferably comprises the following steps: mixing the compound A with the solvent, and then slowly adding dropwise into an anti-solvent; the solvent is selected from the group consisting of methanol, tetrahydrofuran and DMSO; the anti-solvent is water; and the volume ratio of the solvent to water is 3:1 to 1:1 (for example, 3:1 or 1:1).

In the preparation method of the crystalline form III, when the scheme 3 is adopted, the preparation method of the crystalline form III preferably comprises the following steps: adding the solution of the compound A and the solvent into aqueous solution A, and crystallizing, wherein the aqueous solution A is the suspension of the crystal seeds of crystalline form III of substance A and water; the solvent is DMSO; the volume ratio of DMSO to water is 1:1 to 1:4 (for example, 1:1, 2:3, 1:2 or 1:4).

The present disclosure also provides a preparation method of the crystalline form V of the compound of formula A, wherein, the preparation method is scheme A or scheme B;
scheme A comprises the following steps: transforming a suspension of the amorphous form of the compound of formula A and a solvent into a crystalline form V of compound A at 20 to 50 °C; the solvent is water or nitrile solvent.
Scheme B comprises the following steps: volatilizing the solvent in a solution of the compound of formula A and a solvent into a crystalline form V of compound A; the solvent is alcohol solvent.

In scheme A, the mass volume ratio of the amorphous form of compound A to the solvent is preferably 3.0mg/mL or 50mg/mL.

In scheme A, the solvent is preferably water or acetonitrile.

In scheme A, the temperature of crystal transformation is preferably 50°C.

In scheme B, the solvent is preferably methanol.

In a certain embodiment of the scheme A, the scheme comprises the following steps: transforming a suspension of the amorphous form of compound A and a solvent into a crystalline form V of the compound at 20 to 50 °C; the solvent is water or acetonitrile; the temperature of crystallization is preferably 50 °C, and the mass volume ratio of the amorphous form of compound A to the solvent is 50 mg/mL or 3.0 mg/mL.

In a certain embodiment of the scheme B, the scheme comprises the following steps: volatilizing the solvent in a solution of the compound A and a solvent into a crystalline form V of compound A; the solvent is methanol, the temperature is 50 °C.

In the present disclosure, the amorphous form of the compound of formula A is prepared by the method in patent application CN201911379293.8 (see the examples for details).

A pharmaceutical composition comprising above-mentioned crystalline form (for example, the crystalline form is selected from the group consisting of crystalline form I, crystalline form II, crystalline form III, crystalline form IV, crystalline form V, crystalline form VI, crystalline form VII, crystalline form VIII and crystalline form IX) of the compound represented by formula A or the solvate thereof and/or the above-mentioned crystalline form III of substance A, and pharmaceutical excipient. Herein, the dosage of the crystalline form can be a therapeutic effective amount.

The pharmaceutical excipient can be excipient widely used in the art of pharmaceutical production. Excipient is mainly used to provide a safe, stable and functional pharmaceutical composition, and can also provide a method for allowing a subject to receive the administration of an active ingredient to dissolve at a desired rate, or facilitating effective absorption of the active ingredient after the subject receives the administration of the composition. The pharmaceutical excipient can be inert filler or provide certain functions, for example stabilizing the overall pH value of the composition or preventing the degradation of active ingredients of the composition. The pharmaceutical excipient can be selected from the group consisting of the following excipient: binder, suspending agent, emulsifier, diluent, filler, granulating agent, adhesive, disintegrating agent, lubricant, anti-sticking agent, glidant, wetting agent, gelling agent, absorption delaying agent, dissolution inhibitor, enhancer, adsorbent, buffer, chelating agent, preservative, colorant, flavor and sweetener.

Substance that can be used as the pharmaceutically acceptable excipient includes, but is not limited to, ion exchanger, aluminum, aluminum stearate, lecithin, serum protein, for example human serum protein, buffer substance for example phosphate, glycine, sorbic acid, potassium sorbate, partial glyceride mixture of saturated vegetable fatty acid, water, salt or electrolyte, for example protamine sulfate, disodium hydrogen phosphate, potassium hydrogen phosphate, sodium chloride, zinc salt, colloidal silicon, magnesium trisilicate, polyvinylpyrrolidone, polyacrylate, wax, polyethylene-polyoxypropylene-blocking polymer, lanolin, sugar, for example lactose, glucose and sucrose; starch for example corn starch and potato starch and the derivative thereof for example sodium carboxymethyl cellulose, ethyl cellulose and cellulose acetate; tree rubber powder; malt; gelatin; talc powder; for example cocoa butter and suppository wax; for example peanut oil, cottonseed oil, safflower oil, sesame oil, olive oil, corn oil and soybean oil; diol compound, for example propylene glycol and polyethylene glycol; Such as ethyl oleate and ethyl laurate; agar; buffering agent such as magnesium hydroxide and aluminum hydroxide; alginic acid; pyrogen-free water; isotonic salt; ringer's solution; ethanol, phosphoric acid buffer solution, and other nontoxic suitable lubricants for example sodium lauryl sulfate and magnesium stearate, colorant, release agent, coating material, sweetener, flavoring agent and spice, preservative and antioxidant.

The pharmaceutical composition of the present disclosure can be prepared by any methods known to those skilled in the art according to the disclosure. For example, conventional mixing, dissolving, granulating, emulsifying, attenuating, encapsulating, entrapping or lyophilizing processes.

The present disclosure provides a use of the above-mentioned crystalline form (for example, the crystalline form is selected from the group consisting of crystalline form I, crystalline form II, crystalline form III, crystalline form IV, crystalline form V, crystalline form VI, crystalline form VII, crystalline form VIII and crystalline form IX) of the compound represented by formula A or the solvate thereof, the above-mentioned crystalline form III of the substance A in the manufacture of a P2X3 inhibitor. In the use, the P2X3 inhibitor can be used in mammalian organisms; it can also be used *in vitro,* mainly for experimental purposes, for example, providing comparison as a standard sample or a control sample, or preparing a kit according to the conventional method in the art, so as to provide a rapid detection of the inhibition effect of P2X3.

The present disclosure provides a use of the above-mentioned crystalline form (for example, the crystalline form is selected from the group consisting of crystalline form I, crystalline form II, crystalline form III, crystalline form IV, crystalline form V, crystalline form VI, crystalline form VII, crystalline form VIII and crystalline form IX) of the compound represented by formula A or the solvate thereof in the manufacture of a medicament, the medicament is a medicament used to protect, deal with, treat, or alleviate at least part of P2X3-mediated or activity-related diseases of animals; alternatively, the medicament is a medicament used to treat pain, urinary tract disease, or respiratory disease.

The present disclosure provides a use of the above-mentioned crystalline form (for example, the crystalline form is selected from the group consisting of crystalline form I, crystalline form II, crystalline form III, crystalline form IV, crystalline form V, crystalline form VI, crystalline form VII, crystalline form VIII and crystalline form IX) of the compound represented by formula A or the solvate thereof, the above-mentioned crystalline form III of the substance A or the above-mentioned pharmaceutical composition for protecting, dealing with, treating or alleviating at least part of P2X3-mediated or activity related disease of animal (such as human ). The disease includes, but is not limited to, respiratory disease, cough, chronic cough, idiopathic pulmonary fibrosis, chronic pulmonary obstruction, asthma, pain, urinary incontinence, autoimmune disease, overactive bladder, dysuria, inflammation, alzheimer's disease, parkinson's, sleep disorders, epilepsy, mental illness, arthritis, neurodegeneration, traumatic brain injury, myocardial infarction, rheumatoid arthritis, stroke, thrombosis, atherosclerosis, colon syndrome, inflammatory bowel disease, digestive tract disease, gastric bowel dysfunction, respiratory failure, sexual dysfunction, cardiovascular disease, heart failure, hypertension, urinary incontinence, cystitis, arthritis, endometriosis, blood disease, musculoskeletal and connective tissue developmental disorders, and systemic disorders.

In some embodiments, the disease includes pain; the pain includes but is not limited to inflammatory pain, surgical pain, visceral pain, dental pain, premenstrual pain, central pain, pain due to burns, migraine and cluster headache.

In some embodiments, the disease includes urinary tract disease; the urinary tract disease includes urinary incontinence, overactive bladder, dysuria and cystitis.

In some embodiments, the disease includes respiratory disease, the respiratory disease includes but is not limited to breathing disorder comprising idiopathic pulmonary fibrosis, chronic obstructive pulmonary disease, asthma, bronchospasm and chronic cough.

The present disclosure provides a method for protecting, dealing with, treating, or alleviating at least part of P2X3-mediated or activity related disease of animals (such as humans), comprising administrating the above-mentioned crystalline form (e.g., the crystalline form is selected from the group consisting of crystalline form I, crystalline form II, crystalline form III, crystalline form IV, crystalline form V, crystalline form VI, crystalline form VII, crystalline form VIII and crystalline form IX) of the compound represented by formula A or the solvate thereof, the above-mentioned crystalline form III of substance A, and the above-mentioned pharmaceutical composition. The disease includes, but is not limited to, respiratory disease, cough, chronic cough, idiopathic pulmonary fibrosis, chronic pulmonary obstruction, asthma, pain, urinary incontinence, autoimmune disease, overactive bladder, dysuria, inflammation, alzheimer's disease, parkinson's, sleep disorders, epilepsy, mental illness, arthritis, neurodegeneration, traumatic brain injury, myocardial infarction, rheumatoid arthritis, stroke, thrombosis, atherosclerosis, colon syndrome, inflammatory bowel disease, digestive tract disease, gastric bowel dysfunction, respiratory failure, sexual dysfunction, cardiovascular disease, heart failure, hypertension, urinary incontinence, cystitis, arthritis, endometriosis, blood disease, musculoskeletal and connective tissue developmental disorders, and systemic disorders.

In some embodiments, the disease includes pain; the pain includes but is not limited to inflammatory pain, surgical pain, visceral pain, dental pain, premenstrual pain, central pain, pain due to burns, migraine and cluster headache.

In some embodiments, the disease includes urinary tract disease; the urinary tract disease includes urinary incontinence, overactive bladder, dysuria and cystitis.

In some embodiments, the disease includes respiratory disease, the respiratory disease includes but is not limited to breathing disorder comprising idiopathic pulmonary fibrosis, chronic obstructive pulmonary disease, asthma, bronchospasm and chronic cough.

The above-mentioned pharmaceutical composition is characterized in that the side effects of taste disorders associated with treatment are reduced by administering the pharmaceutical composition.

On the basis of conforming to the common sense in the field, the above-mentioned preferred conditions can be arbitrarily combined to obtain the preferred examples of the present disclosure.

The reagents and raw materials used in the invention are commercially available.

The positive progressive effects of the present disclosure are:
1. The crystalline form of the heterocyclic compound has not been reported in the prior art, and many new crystalline forms of the compound have been found in this disclosure for the first time. Through a large number of experiments and screening, the present disclosure firstly prepared to obtain crystalline form I, crystalline form II, crystalline form III, crystalline form IV, crystalline form V, crystalline form VI, crystalline form VII, crystalline form VIII or crystalline form IX, and used as candidates.
2. Part of the crystalline forms prepared by the present disclosure have good stability, are convenient for storage, can avoid the risk of crystalline transformation during drug development or preparation process, avoid the change of bioavailability and efficacy, which can be developed into a dosage form suitable for clinical use with strong economic value.
3. The present disclosure also provides preparation methods for the crystalline form of crystalline form I, crystalline form II, crystalline form III, crystalline form IV, crystalline form V, crystalline form VI, crystalline form VII, crystalline form VIII, the salt of crystalline form IX or solvate thereof, the methods are simple and easy to operate, reproducible, are not easy to remain solvent, environmentally friendly, and suitable for different large-scale production.

### BRIEF DESCRIPTION OF THE DRAWINGS

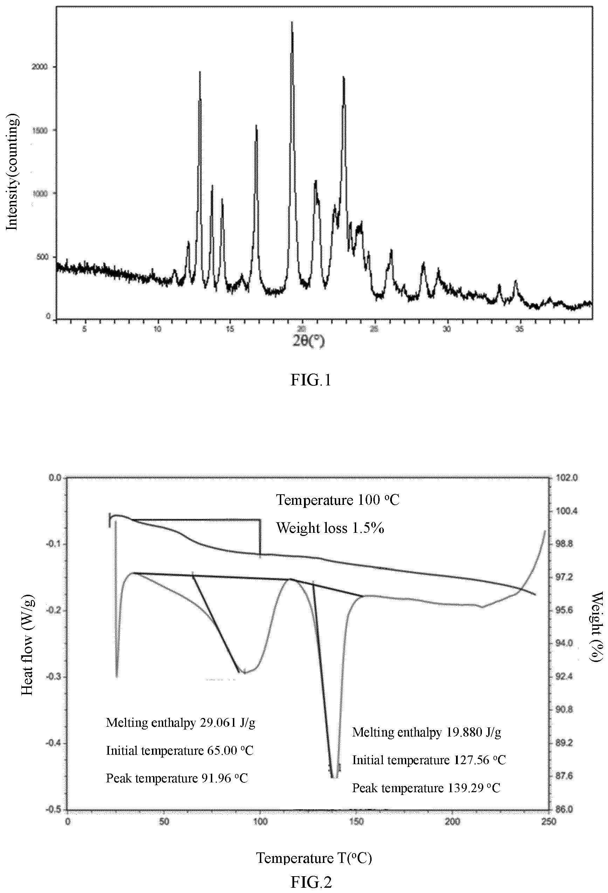

Fig. 1 is the XRPD pattern of the crystalline form III;
Fig.2 is the TGA/ DSC superimposition pattern of the crystalline form III;

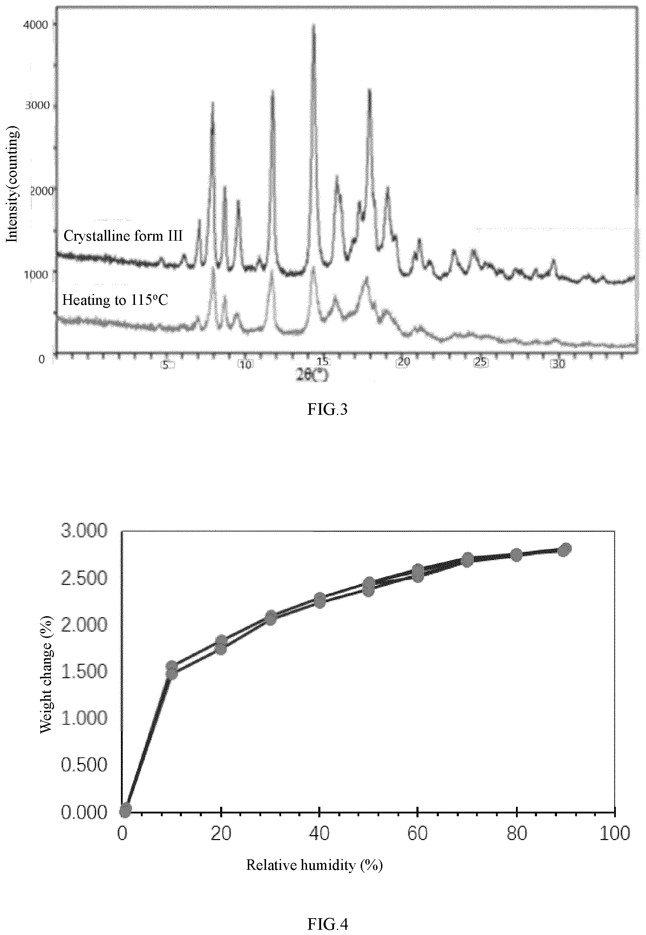

Fig.3 is the XRPD superimposition pattern of the crystalline form III before and after heating to dehydration;
Fig.4 is the DVS pattern of the crystalline form III;

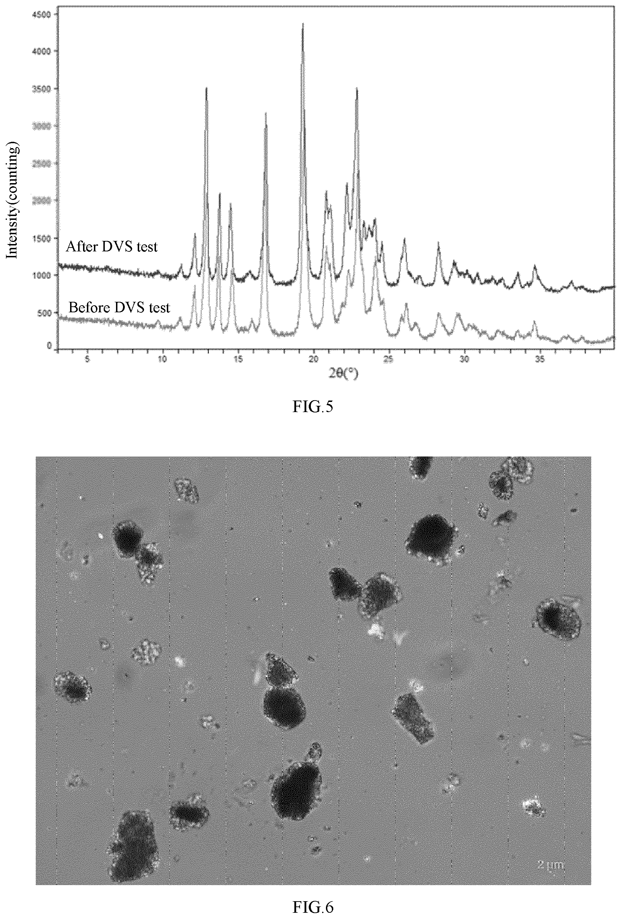

Fig. 5 is the XRPD superimposition pattern before and after DVS test of the crystalline form III;
Fig.6 is the PLM pattern of the crystalline form III;
Fig.7 is the XRPD pattern of the crystalline form V;
Fig.8 is the TGA/ DSC superimposition pattern of the crystalline form V;
Fig.9 is the XRPD superimposition pattern before and after DVS test of the crystalline form V;
Fig. 10 is the DVS pattern of the crystalline form V;
Fig. 11 is the PLM pattern of the crystalline form V;
Fig. 12 is the XRPD pattern of the crystalline form I;
Fig. 13 is the TGA/ DSC superimposition pattern of the crystalline form I;
Fig. 14 is the DVS pattern of the crystalline form I;
Fig. 15 is the XPRD pattern of the crystalline form I after moisture absorption;
Fig. 16 is the XRPD superimposition pattern before and after DVS test of the crystalline form I;
Fig. 17 is the PLM pattern of the crystalline form I;
Fig. 18 is the XRPD pattern of the crystalline form II;
Fig. 19 is the HNMR pattern of residual MTBE of the crystalline form II;
Fig.20 is the TGA/ DSC superimposition pattern of the crystalline form II;
Fig.21 is the PLM pattern of the crystalline form II;
Fig.22 is the XRPD pattern of the crystalline form IV;
Fig.23 is the TGA/ DSC superimposition pattern of the crystalline form IV;
Fig.24 is the XRPD superimposition pattern dehydration of crystalline form IV;
Fig.25 is the PLM pattern of the crystalline form IV;
Fig.26 is the XRPD pattern of the crystalline form VI;
Fig.27 is the XRPD superimposition pattern of the crystalline form VI;
Fig.28 is the XRPD pattern of the crystalline form VII;
Fig.29 is the HNMR pattern of residual ethylene glycol of the crystalline form VII;
Fig.30 is the TGA/ DSC superimposition pattern of the crystalline form VII;
Fig.31 is the XRPD pattern of the crystalline form VIII;
Fig.32 is the TGA/ DSC superimposition pattern of the crystalline form VIII;
Fig.33 is the HNMR pattern of the crystalline form VIII and raw material;
Fig.34 is the XRPD superimposition pattern of the crystalline form VIII and the sample before drying;
Fig.3 5 is the XRPD pattern of the crystalline form IX;
Fig.36 is the TGA/ DSC superimposition pattern of the crystalline form IX;

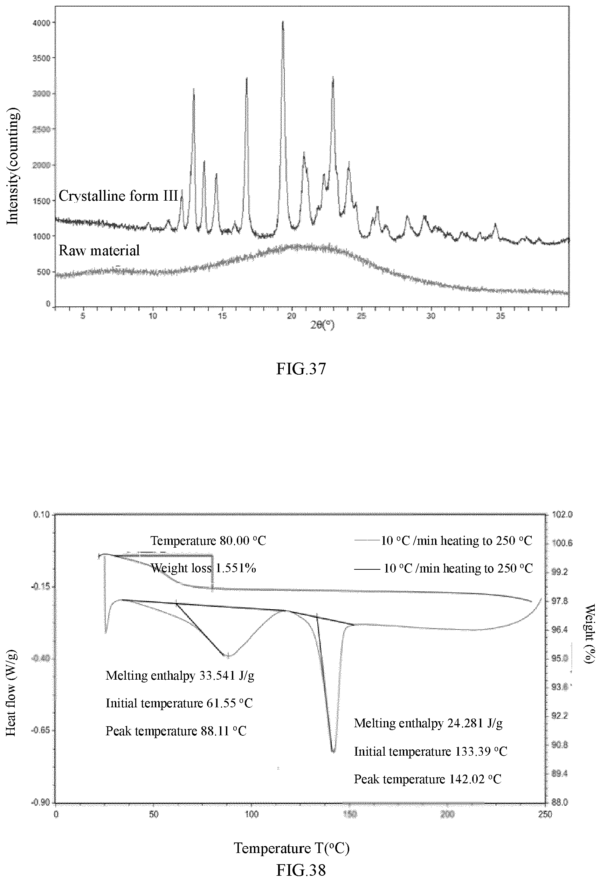

Fig.37 is the XRPD superimposition pattern of the crystalline form III obtained from an amorphous sample;
Fig.38 is the DSC&TGA superimposition pattern of the crystalline form III obtained from an amorphous sample;

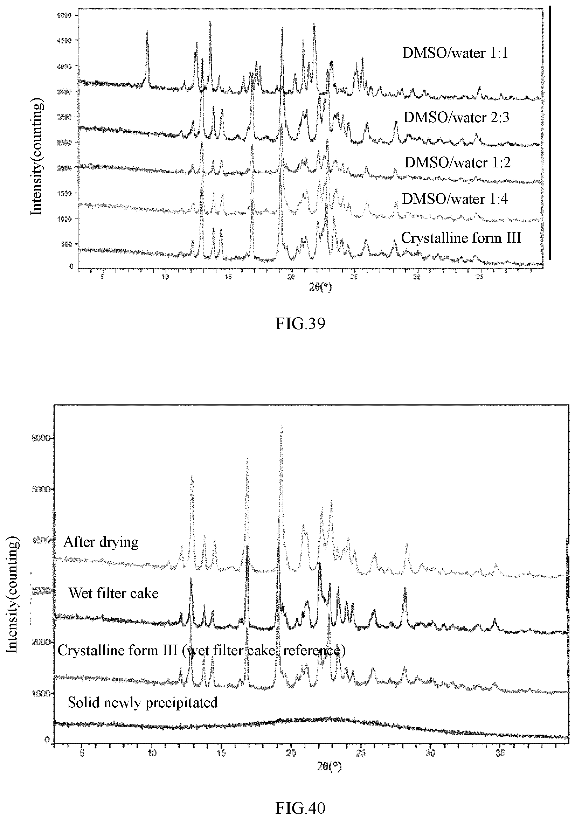

Fig.39 is the XRPD superimposition pattern of the crystalline form III slurried in mixed solvent with different volume ratios;
Fig.40 is the XRPD superimposition diagram of 200 mg grade of crystalline form III;

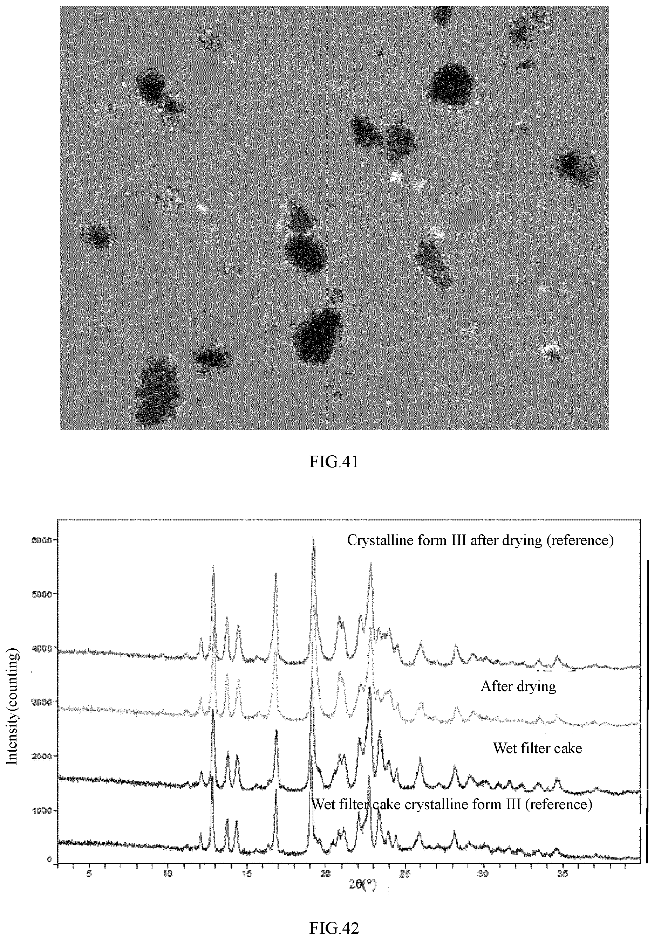

Fig.41 is the PLM pattern of 200 mg grade of crystalline form III;
Fig.42 is the XRPD superimposition pattern of the crystalline form III (3 g);

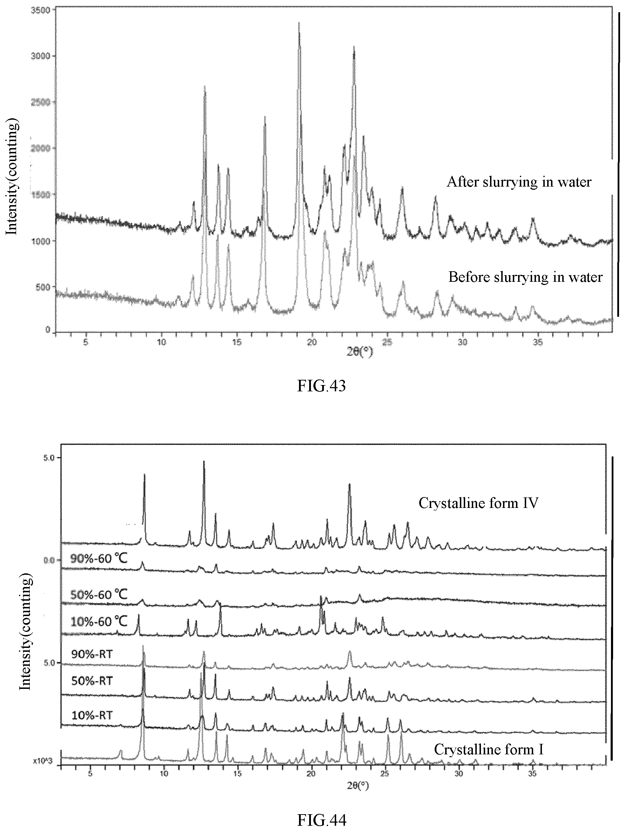

Fig.43 is the XRPD superimposition pattern dehydration of crystalline form III before and after slurrying in water at room temperature;
Fig.44 is the water activity test result of crystalline form I;

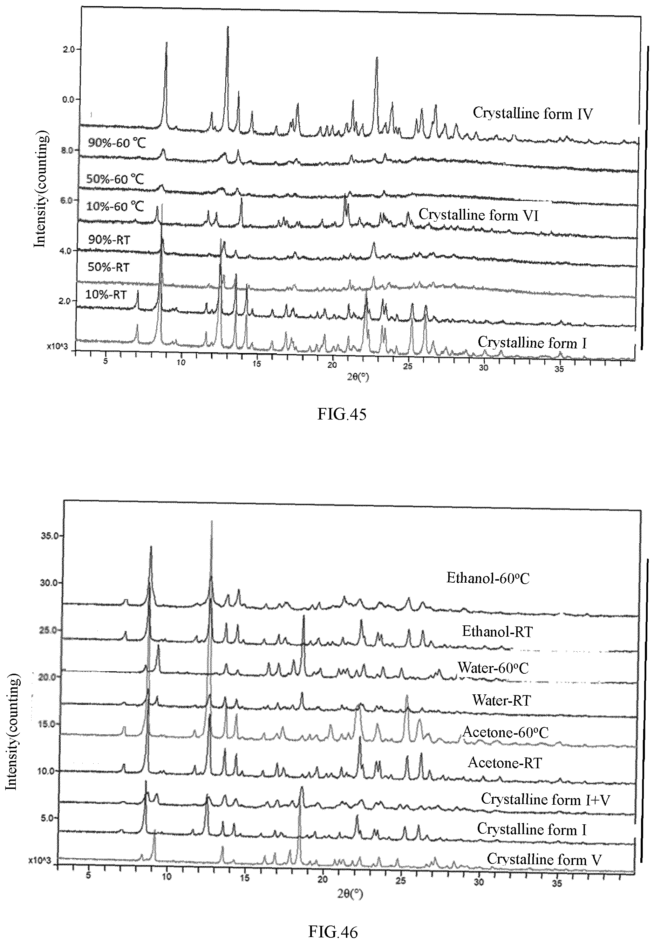

Fig.45 is the water activity test result of crystalline form V;
Fig.46 is the XRPD superimposition pattern of competitive slurrying experiment of crystalline form I and crystalline form V;

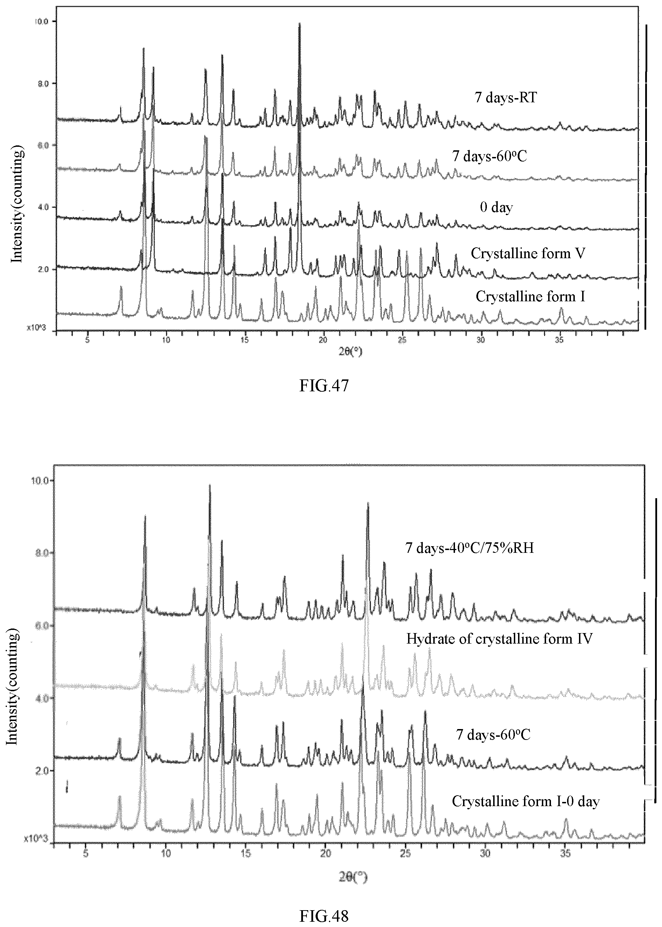

Fig.47 is the results of stability data of crystalline forms I and V in solid state;
Fig.48 is the XRPD superimposition pattern of the stability test sample of crystalline form I;

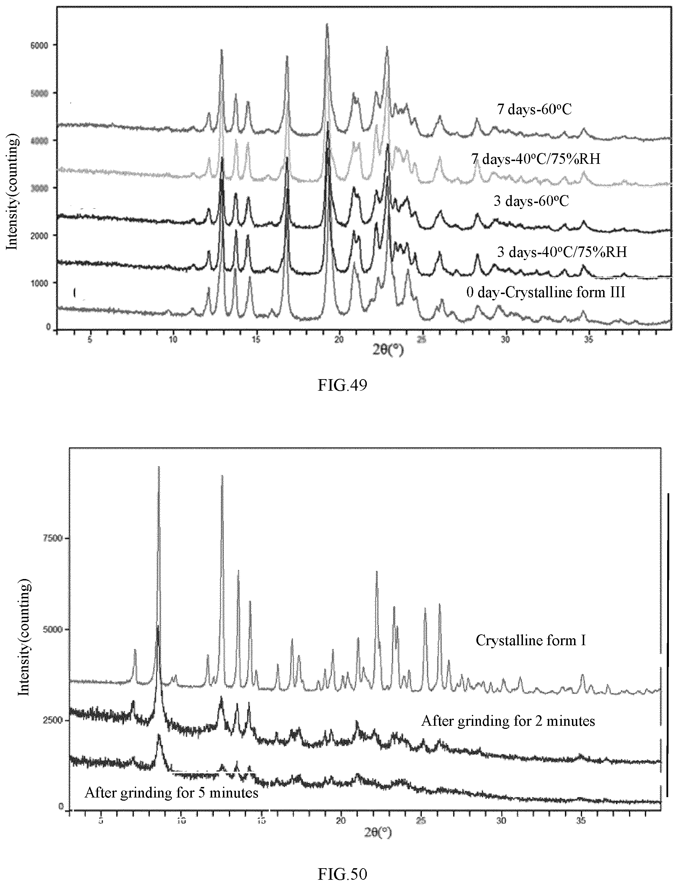

Fig.49 is the XRPD superimposition pattern of the stability test sample of crystalline form III;
Fig.50 is the XRPD superimposition pattern before and after grinding test of the crystalline form I;

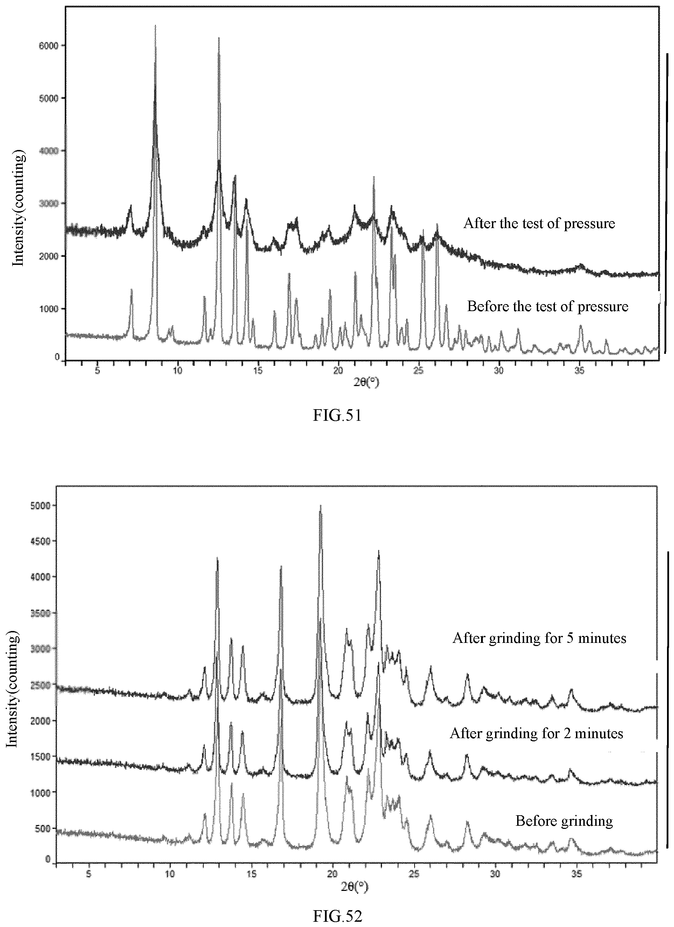

Fig.51 is the XRPD superimposition pattern before and after pressure test of the crystalline form I;
Fig.52 is the XRPD superimposition pattern before and after grinding test of the crystalline form III;

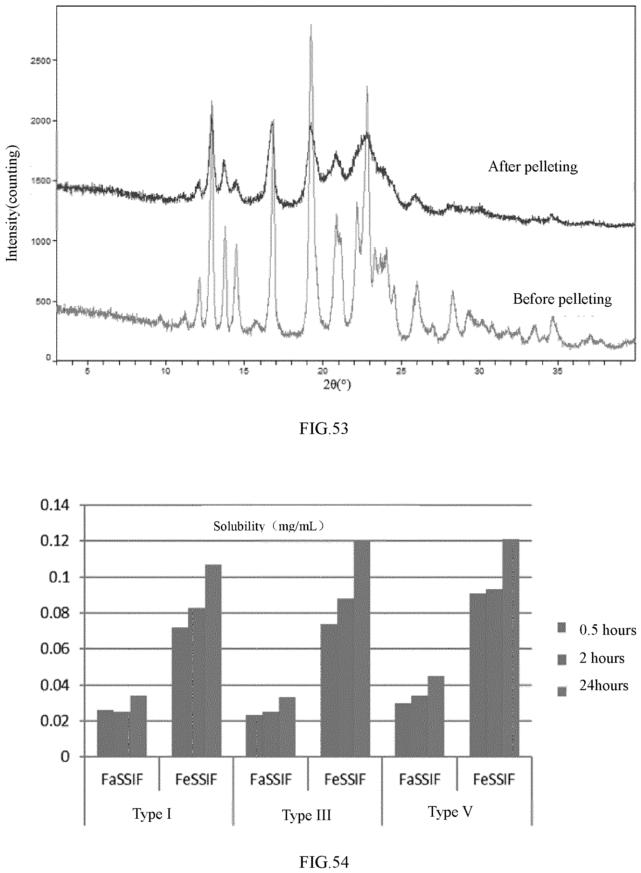

Fig.53 is the XRPD superimposition pattern before and after pressure test of the crystalline form III;
Fig.54 is the solubility data pattern of crystalline forms I, III and V;

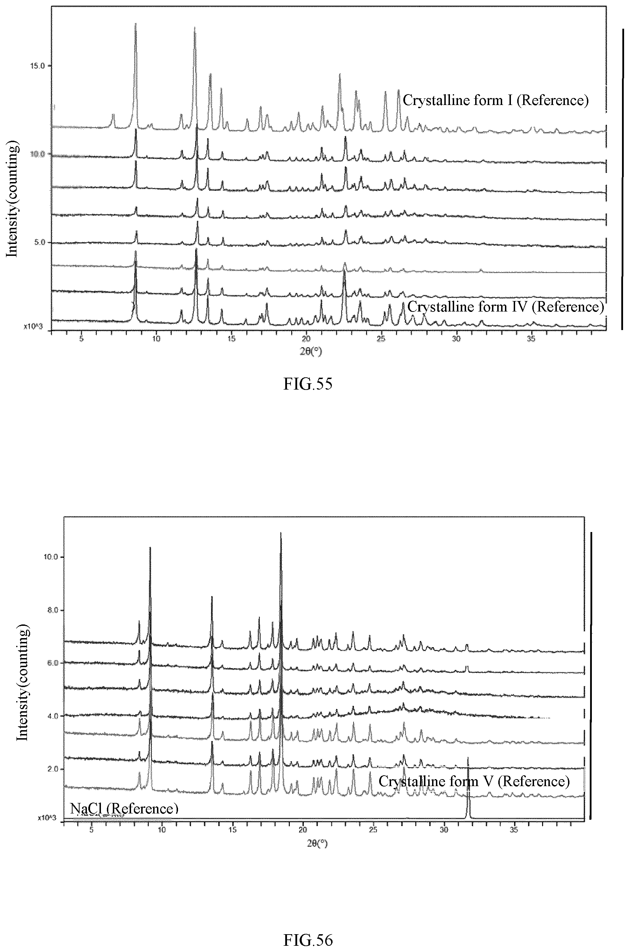

Fig.55 is the XRPD superimposition pattern of the remaining solid in solubility test of crystalline form I;
Fig. 56 is the XRPD superimposition pattern of the remaining solid in solubility test of the crystalline form V;

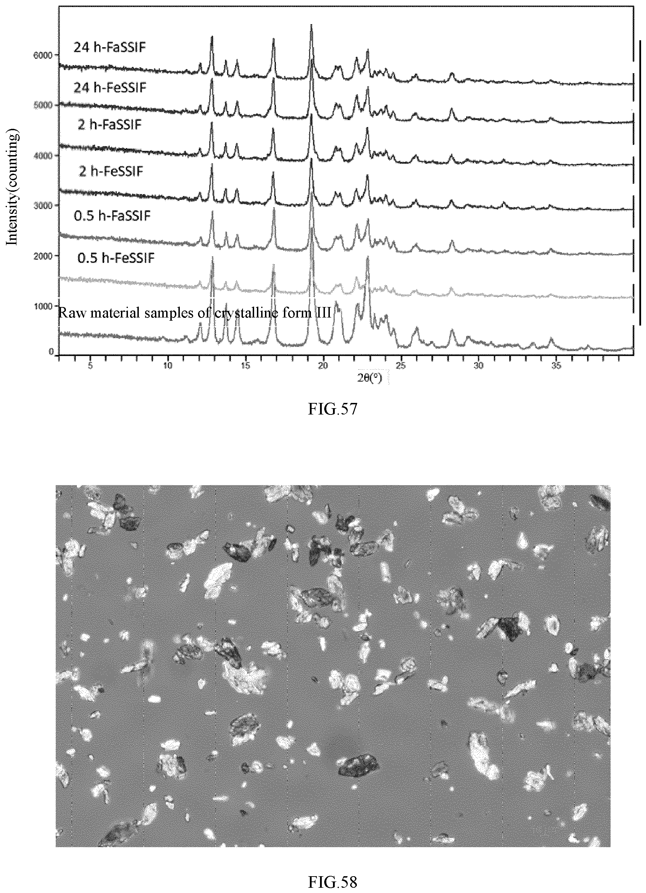

Fig. 57 is the XRPD superimposition pattern of the remaining solid in solubility test of the crystalline form III;
Fig.58 is the PLM pattern of an amorphous sample;
Fig.59 is the DSC&TGA superimposition pattern of the amorphous sample;
Fig.60 is the ¹HNMR pattern of the crystalline form IX;

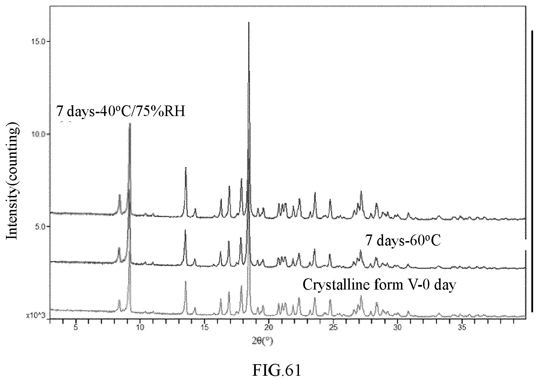

Fig.61 is the XRPD superimposition pattern of the stability test sample of crystalline form V

### DETAILED DESCRIPTION OF THE PREFERRED EMBODIMENT

The invention is further described below by way of embodiments, but the invention is not thereby limited to the scope of the described embodiments. The experimental methods not specified in the specific conditions in the following examples are selected according to the conventional methods and conditions, or according to the commodity instructions.

The present disclosure will be further described in detail below with reference to the specific embodiments. In order to further understand the present disclosure, these embodiments are used to illustrate the basic principles, main features and advantages of the present disclosure, and the present disclosure is not limited by the following embodiments. The implementation conditions adopted in the examples can be further adjusted according to the specific requirements, and the implementation conditions not indicated are usually those in routine experiments.

In the following embodiments, the experimental method is typically completed in accordance with conventional conditions or conventional test conditions, and the compound may be obtained by organic synthesis or by commercially available methods. The compound used in the following embodiment is obtained by a commercially available method, and the purity reaches 99%.

The abbreviations used in the present disclosure are explained as follows:
XPRD-X-ray powder diffraction
TGA-Thermogravimetric analysis
DSC-Differential Scanning Calorimetry
DVS-Dynamic Vapor Sorption
PLM-Polarized light microscopy

The test conditions are as follows:

### XRPD

The solids were characterized using an X-ray powder diffractometer (Brooke D8 advance or D2 Phase).
Scanning angle: 3° (2θ) - 40° (2θ).
Step size: 0.02° (2θ).
Scan speed: 0.3 sec/step (D8), 0.2 sec/step (D2).
Tube voltage: 40KV (D8), 30KV (D2).
Tube current: 40 mA (D8), 10 mA (D2).
Rotation: On.
Sample tray: Zero background sample tray.

### TGA

The solid samples were analyzed by thermogravimetric analysis using TA Instrument thermogravimetric analysis Q500 or Discovery TGA 55. After balancing the sample tray, the sample was hung on the hanging wire and put on the stove. After stabilization, the sample was heated at a rate of 10°C/min to different end temperatures.

### DSC

The solid samples were analyzed by DSC using TA Instrument Differential Scanning Calorimeter Q200 and Discovery DSC 250. The samples were weighed and the values were recorded, and then the samples were placed in the sample chamber. The sample was heated at a rate of 10°C/min from 25°C to different end temperatures.

### DVS

The solid was analyzed by DVS using IGA sorp dynamic vapor sorption instrument.
Temperature: 25 °C.
Gas flow: 250 mL/min.
Scan cycle: 2.
Minimum test time: 30 min.
Maximum test time: 2 hours.
Waiting for balance: 98%.

### PLM

The sample was observed using a Nikon Eclipse LV100N POL polarizing microscope.

### Embodiment 1: Preparation of the compound of formula A

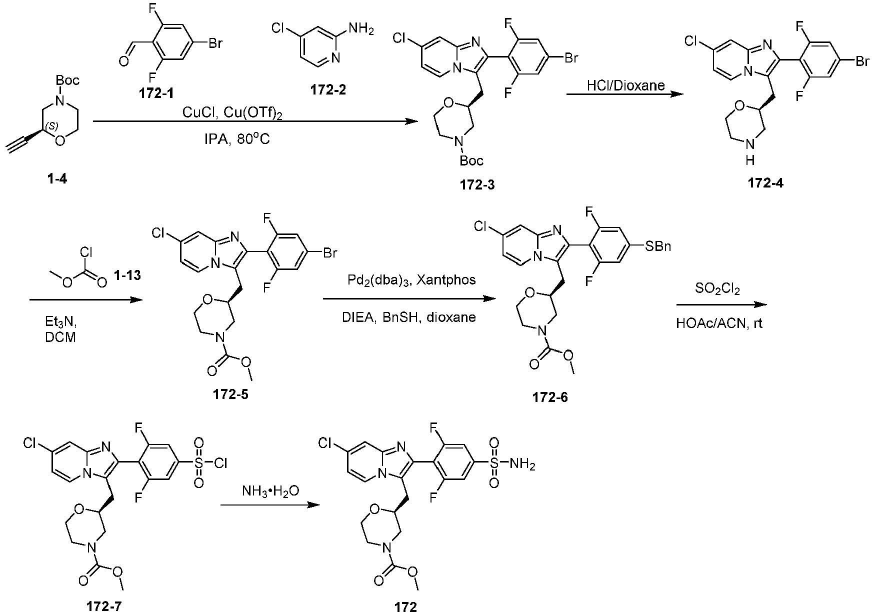

### Step 1 Preparation of tert-butyl (S)-2-((2-(4-bromo-2,6-difluorophenyl)-7-chloroimidazo[1,2-a]pyridin-3-yl)methyl)morpholine-4-carboxylate

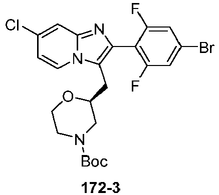

*tert*-Butyl (S)-2-ethynylmorpholine-4-carboxylate (3.1 g, 1.0 eq, intermediate 1-4), 4-bromo-2,6-difluorobenzaldehyde (2.76 g, 1.0 eq, compound 172-1), 4-chloropyridine-2-amine (1.61 g, 1.0 eq, compound 172-2), CuCl (0.37 g, 0.3 eq), Cu(OTf)₂ (1.36 g, 0.3 eq) and isopropanol (50 mL) were added in a 100 mL round-bottom flask in turn, and the reaction solution was replaced with nitrogen for 3 times, and then heated overnight in an oil bath at 80 °C, the mixture was detected with TLC and the TLC showed that the raw compound 172-2 disappeared. Isopropanol was evaporated to dryness by rotary evaporation, and the mixture was extracted with EA and ammonia water in turn, and then the EA phase was taken, washed with saturated brine and citric acid, and then dried over anhydrous sodium sulfate, the mixture was evaporated to dryness by rotary evaporation and purified with column to obtain intermediate 172-3 as white solid (3.0 g, purity 78%). LC-MS: [M+H]⁺ =542.2.

### Step 2 Preparation of (S)-2-((2-(4-bromo-2,6-difluorophenyl)-7-chloroimidazo[1,2-a]pyridin-3-yl)methyl)morpholine

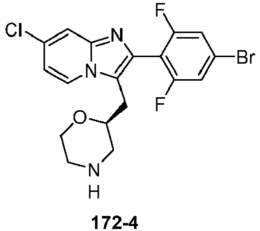

Intermediate 172-3 (2.67 g) was dissolved in dichloromethane (24 mL), and dioxane hydrochloride (24 mL) was added thereto, and the mixture was stirred at room temperature for 1.0 hour, and the mixture was detected with LC-MS, and the LC-MS showed that the reaction was finished. The reaction solution was evaporated to dryness by rotary evaporation, and added with water (15 mL) and dichloromethane (15 mL), and then water phase was removed after extraction, and the pH of the water phase was adjusted to be weakly alkaline (pH=8 to 9) with aqueous sodium bicarbonate solution. The dichloromethane phase was separated, and the aqueous phase was extracted with dichloromethane (10 mL×2). The dichloromethane phase was combined, washed with saturated brine, and evaporated to dryness by rotary evaporation to obtain intermediate 172-4 as white solid (1.70 g, purity 88.6%). LC-MS: [M+H]⁺ =442.1.

### Step 3 Preparation of methyl (S)-2-((2-(4-bromo-2,6-difluorophenyl)-7-chloroimidazo[1,2-a]pyridin-3-yl)methyl)morpholine-4-carboxylate

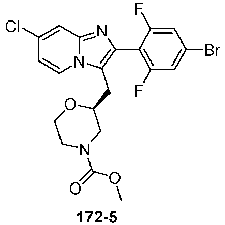

Intermediate 172-4 (1.4 g, 1.0 eq) was dissolved in dichloromethane (10 mL), then triethylamine (480 mg, 1.5 eq) and methyl chloroacetate (388 mg, 1.3 eq) were added thereto dropwise. LC-MS showed product formation after 1.0 hour of the reaction. After the reaction was completed, water (10 mL) was added and the mixture was stirred for 30min, and then the dichloromethane phase was separated, and the aqueous phase was extracted with dichloromethane (10 mL×2). The dichloromethane phase was combined, washed with saturated sodium chloride, dried over anhydrous sodium sulfate, and evaporated to dryness by rotary evaporation, and the mixture was purified by column to obtain intermediate 172-5 as white solid (1.01 g, purity 93.02%). LC-MS: [M+H]⁺ =499.8.

### Step 4 Preparation of methyl (S)-2-((2-(4-(benzylthio)-2,6-difluorophenyl)-7-chloroimidazo[1,2-a]pyridin-3-yl)methyl)morpholine-4-carboxylate

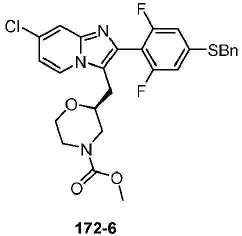

Intermediate 172-5 (0.73 g, 1.0 eq) was dissolved in dioxane (4 mL), then BnSH (0.24 g, 1.3 eq), Pd₂(dba)₃ (0.04 g, 0.03 eq), Xantphos (0.04 g, 0.05 eq) and DIEA (0.60 g, 3.0 eq) were added thereto, and the mixture was replaced with N₂ for three times and reacted overnight at 80 °C. LCMS monitored that the raw materials disappear completely. The reaction solution was added with dichloromethane (10 mL) and water (10 mL), and then the dichloromethane phase was separated, and the aqueous phase was extracted with dichloromethane (10 mL×2). The dichloromethane phase was combined, washed with saturated sodium chloride, dried over anhydrous sodium sulfate, and evaporated to dryness by rotary evaporation, and the mixture was purified by column to obtain intermediate 172-6 as white solid (0.82 g, purity 91.53%). LC-MS: [M+H]⁺ =544.2.

### Step 5 Preparation of methyl (S)-2-((7-chloro-2-(4-(chlorosulfonyl)-2,6-difluorophenyl)imidazo[1,2-a]pyridin-3-yl)methyl)morpholine-4-carboxylate

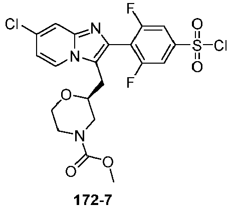

Intermediate 172-6 (510 mg) was added to a reaction flask, and acetonitrile (3 mL) was added to dissolve, and then glacial acetic acid (281 mg, 5.0 eq) was added thereto, and SO₂Cl₂ (506 mg, 4.0 eq) was added dropwise under an ice bath. And then the mixture was reacted at 0°C for 1 hour. LCMS showed that the raw material disappeared and intermediate 172-7 was formed. The reaction solution was directly used for the next reaction without post-treatment.

### Step 6 Preparation of methyl (S)-2-((7-chloro-2-(2,6-difluoro-4-sulfamoylphenyl)imidazo[1,2-a]pyridin-3-yl)methyl)morpholine-4-carboxylate

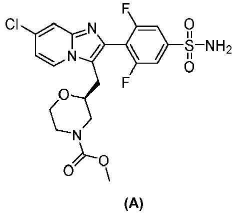

At 0°C, ammonia water (2 mL) diluted with acetonitrile (1 mL) was added dropwise to the above reaction solution, and the mixture was reacted at room temperature for 0.5 hours. LCMS showed that the raw material disappeared completely and the target product was formed. The reaction solution was extracted with water and ethyl acetate for 2 times, and washed with brine solution, and then dried over anhydrous sodium sulfate, the mixture was concentrated and separated and then purified by C18 column (water/acetonitrile, Rt=22.5 minutes). Compound A, white solid, was obtained in the amorphous state. (185 mg, purity 99.74%). LC-MS: [M+H]⁺ =501.1.

¹H NMR (400 MHz, DMSO-*d*₆) δ = 8.11 (d, J=7.4, 1H), 7.29 (d, J=1.6, 1H), 7.22 (s, 2H), 7.14 (d, J=6.6, 2H), 6.60 (dd, J=7.4, 2.1, 1H), 3.33 (d, J=12.8, 1H), 3.13 (d, J=11.3, 2H), 3.07 (s, 3H), 2.97 (d, J=7.8, 1H), 2.77 - 2.69 (m, 1H), 2.69 - 2.61 (m, 1H), 2.53 (dd, J=15.5, 8.3, 1H).

### Embodiment 2: Post-treatment of raw materials

A total of 11.1g of compound A was prepared by referring to embodiment 1, and 20 mL of acetone was added, and the mixture was refluxed at 65°C (under the protection of nitrogen) for 2.0 hours, acetone was directly evaporated to dryness by rotary evaporation, and vacuum dried at 40°C for 12 hours, and then nuclear magnetic resonance showed that there was about 1% acetone residue. After vacuum drying at 80°C for 12 hours, ¹H NMR showed that there was still acetone residue. 5.3g of the residue was dried in vacuum at 80°C for 12 hours again, and the nuclear magnetic resonance showed that there was still acetone residue. The batch of products was added to acetonitrile (16 mL), and refluxed at 85°C (the protection of nitrogen) for 2.0 hours, and then acetonitrile was directly evaporated to dryness by rotary evaporation, and then vacuum dried at 80°C for 12 hours, and the nuclear magnetic resonance showed qualified without solvent residue, and put in storage for 5.2g. The purity of the product is 99.29% as white powder.

The PLM pattern and XRPD results showed that the raw material was an irregularly shaped crystal of 10 to 50 µm, with a general crystallinity and an amorphous state. As shown in Fig.37, the DSC pattern showed that the raw material has two connected endothermic peaks between about 150 to 180 °C, and the peak temperatures are 164±2 °C and 173 °C±2 °C, respectively, as shown in Fig.38, the TGA pattern showed that the sample was substantially weightless before 230 °C.

### Embodiment 3: preparation and characterization of amorphous form of compound of formula A

3.1 Compound A was dissolved in a certain amount of THF and concentrated under reduced pressure to dry, to obtain an amorphous sample. The XRPD characterization is as shown in Fig.37.

Embodiment 4: preparation and characterization of crystalline form I of the compound of formula A
4.1 Nine kinds of solvents were selected: EtOH, IPA, NBA, MEK, ACN, acetone, EA, IPAc, Hept, and the mixture was suspended and slurried at room temperature, the concentration of slurring is 60mg/mL, to obtain crystalline form I;
4.2 Six kinds of solvents were selected: IPA, NBA, MEK, acetone, Tol, EA, and the mixture was suspended and slurried at 50°C, the concentration of slurring is 100mg/mL, to obtain crystalline form I;
4.3 Solvent selection: IPAc, the mixture was suspended and slurried at 50°C, the concentration of slurring is 50 mg/mL, to obtain crystalline form I;
4.4 Crystalline form I can be obtained by slowly cooling and crystallizing in methanol and ethanol at the temperature of 50°C to RT;
4.5 Tetrahydrofuran was used as a good solvent to dissolve the sample to form a sample solution with a certain concentration, and Tol, Hept or water were slowly added to the sample solution dropwise as anti-solvents, with the volume ratio of 1:10, respectively, so as to improve the supersaturation and precipitate the solid, to obtain crystalline form I;
4.6 EtOH was selected as the solvent for volatile crystallization test to obtain crystalline form I.

The XRPD of crystalline form I represented by 2θ angle is as shown in Fig. 12. In DSC pattern of the crystalline form I, there is an endothermic peak at 152 °C, and the melting enthalpy is 44±2J/g. In TGA pattern of the crystalline form I, there is no weight loss in the temperature range of RT to 230°C. TGA and the DSC pattern are as shown in Fig.13; Combined with DSC and TGA pattern, it can be seen that the product is an anhydrous crystalline form. The DVS pattern of the crystalline form I is as shown in Fig. 14, and when the relative humidity is greater than 40%, the weight of the crystalline form I increases sharply. When the relative humidity drops to 40%, all the absorbed water is discharged. In the XRPD pattern of the crystalline form I after moisture absorption, the crystalline form I absorbs moisture and is transformed into a hydrate crystalline form IV under high humidity environment, which is then dried in a vacuum oven at 30°C and then transformed into the initial crystalline form I, and the XRPD pattern after moisture absorption is as shown in Fig. 15. It can be seen that combined with the DVS test results (Fig. 14), when the ambient relative humidity is higher than 40%, crystalline form I rapidly absorbs moisture and is transformed into hydrate; and when the relative humidity is less than 50%, the adsorbed water is rapidly removed and transformed into crystalline form I. That is, the transformation between crystalline forms I and IV is reversible. The crystalline form I shows hygroscopicity (6.8%, 80%RH), and the crystalline form remains unchanged after the DVS test, and the XRPD pattern before and after the DVS test are as shown in Fig.16. In the PLM pattern of the crystalline form I, the crystalline form is an irregular crystal of about 5 µm, and the PLM pattern is as shown in Fig.17.

### Embodiment 5: preparation and characterization of crystalline form II of the compound of formula A

5.1 In MTBE, the mixture was suspended and slurried at room temperature to obtain crystalline form II:
5.2 Solvent selection: MTBE, the mixture was suspended and slurried at 50°C, the concentration of slurring was 100 mg/mL, to obtain crystalline form II;
5.3 Tetrahydrofuran was used as a good solvent to dissolve the sample to form a sample solution with a certain concentration, and MTBE were slowly added to the sample solution dropwise as anti-solvents, with the volume ratio of 1:10, so as to improve the supersaturation and precipitate the solid, to obtain crystalline form II.

The X-ray powder diffraction pattern of crystalline form II represented by 2θ angle is as shown in Fig.18. In the nuclear magnetic resonance pattern of crystalline form II, there are residual signals of MTBE at the chemical shifts of 1.10 and 3.08 with the molar ratio of 0.39; the HNMR pattern of residual MTBE is as shown in Fig.19. In TGA pattern of crystalline form II, there is a weight loss of 3.5% in the temperature range of 100 to 160 °C, and there is a weight loss of 2.9% in the range of 160 to 200 °C. In DSC pattern of the crystalline form II, there are two adjacent endothermic peaks, and the TGA and DSC pattern are as shown in Fig.20. This is equivalent to HNMR pattern of the residual MTBE as shown in Fig.19, it can be seen that the product is a MTBE solvate. In PLM pattern of the crystalline form II, the crystalline form is an irregular crystal of about 2 µm, and the polarization microscope pattern is as shown in Fig.21.

### Embodiment 6: preparation and characterization of crystalline form III of the compound of formula A

6.1 Solvent selection: water, the mixture was suspended and slurried at 50°C, the concentration of slurring was 50 mg/mL, to obtain crystalline form III;
6.2 The amorphous sample was suspended and slurried in water to obtain crystalline form III at 40°C; specifically, the sample was dissolved in methanol, filtered to obtain a sample solution, and then evaporated by rotary evaporation to obtain 250 mg of amorphous sample. The mixture was suspended and slurried at room temperature after 20 hours to obtain crystalline form III. Through XRPD, DSC, and TGA characterization tests, the seed of the pure crystalline form III was successfully prepared and used as a subsequent amplification experiment.
6.3 Crystalline form III was prepared by solvent-anti-solvent precipitation crystallization and seeding-induced nucleation by adding crystal seed. Specifically, the sample was dissolved in the mixed solvent with a specific ratio as shown in Table 1 in advance, and filtered to obtain a saturated solution, the crystalline form III was added to the saturated solution as a seed crystal, and then water is slowly added as an anti-solvent to precipitate a solid and induce crystallization. The solid sample obtained by filtration was tested by XRPD. The experimental results are as shown in Table 1 and the results show that crystalline form III is unstable in the above-mentioned system, and changes into solvate or unstable hydrate. Considering the solubility and solvent residue limit, DMSO is the third kind of solvent and the solubility is higher than 100 mg/mL, so DMSO is selected for the next research.

**Table 1 Experimental results of solvent-anti-solvent precipitation crystallization**

| Solvent/anti-solvent | V/V | Temperature(°C) | Experimental process | The experimental results |
|---|---|---|---|---|
| MeOH/water | 3/1 | 40 | Crystalline form III was added to the saturated solution as a seed crystal, and then water was slowly added, and the solid slowly was precipitated | Crystalline form IV Hydrate |
| THF/water | 1/1 | 40-RT | Crystalline form III was added to the saturated solution as a seed crystal, and then water was slowly added to appear oil precipitation, and the mixture was cooled to room temperature, and the solid slowly was precipitated | Crystalline form VIII And then the solid was transformed into crystalline form I after drying. |
| DMSO/Water | 3/1 | 40 | The water was slowly added to appear oil precipitation | N/A |
| DMSO/Water | 1/1 | 40 | Crystalline form III was added to the saturated solution as a seed crystal, and then water was slowly added, and the solid slowly was precipitated | Crystalline form IX DMSO solvate |

6.4 It can be seen from the experimental results of 6.3 above that it is difficult to obtain target crystalline form III by adding anti-solvent to the crystallization method of sample solution with good solvent, so it is considered to prepare the target crystalline form by crystallizing compound A by dropwise addition of samples dissolved in organic solvents in an aqueous solution dispersed with seed crystal. The specific operation method is as follows: firstly, the sample was dissolved in DMSO and filtered to obtain DMSO solution, and then the mixture was added into the aqueous solution with seed crystals dispersed slowly. The precipitated solid was transformed into the target crystalline form III under the induction of seed crystal. As shown in Table 2 and Fig. 1, the stability of crystalline form III in different proportions of DMSO/water mixed solvent systems was investigated to determine the appropriate solvent/anti-solvent ratio to ensure the stable existence of crystalline form III in the system. The experimental results show that crystalline form III can be stable in DMSO/water mixed solvent with water content higher than 60% (volume percentage) without crystal transformation at room temperature. When the water content is less than 60%, crystalline form III is transformed to a DMSO solvate (crystalline form IX).

**Table 2 Stability results of crystalline form III in mixed solvents with different volume ratios**

| Solvent/anti-solvent | V/V | Experimental process | The experimental results |
|---|---|---|---|
| DMSO/Water | 1/1 | Crystalline form III is slurried in a mixed solvent for three days at room temperature | Crystalline form IX DMSO solvate |
| | 2/3 | | Crystalline form III |
| | 1/2 | | |
| | 1/4 | | |

Considering the yield of the target product, the theoretical yields of crystalline form III at the above ratio were calculated to further determine the ratio of mixed solvent. As shown in Table 3, for DMSO/ water ratios of 1:2, 1:3 and 1:4, the theoretical yields are almost the same, and the yields are all above 99%, because the solubility of crystalline form III in these three solvents is relatively small.

**Table 3 Theoretical yields of crystalline form III in DMSO/water mixed solvents with different volume ratios**

| Solvent | Solubility (mg/mL, RT) | Theoretical yield (%) |
|---|---|---|
| DMSO | >200 | / |
| DMSO / Water 1: 2 | about 0.5 | 99.3% |
| DMSO / Water 1: 3 | about 0.28 | 99.4% |
| DMSO / Water 1: 4 | < 0.25 | 99.4% |

### 6.5 Amplification of crystalline form III (200 mg)

At room temperature, the sample was dissolved in DMSO and filtered to obtain DMSO solution, which was slowly added to the water dropwise with the crystal seeds of crystalline form III dispersed at the rate of 25µL/min, and with the increase of the dripping amount of DMSO solution, the solid precipitation was gradually increased (DMSO/ water 1:2, volume ratio). XRPD confirmed (Fig.2) that the solid samples newly precipitated were almost amorphous form, and with the extension of reaction time and the induction of seed crystal, after magnetic stirring at room temperature for 16 hours, all the solid samples were transformed to crystalline form III. It was observed that after the reaction was completed, the mixture was stopped stirring, and the solid quickly settled at the bottom, and the upper liquid was substantially transparent. After PLM observation (Fig.6), it was found that the obtained crystalline form III sample was a crystal of about 2 µm, but it was easy to agglomerate into large particles of 20 to 50 µm, so once the mixture was stopped stirring, the solid was settled due to agglomeration. Therefore, the crystalline form was easy to filter. In summary, 200 mg of crystalline form III samples were successfully prepared by anti-anti-solvent precipitation crystallization, with a yield of 93.0%, and the DMSO residual was 0.1% wt in ¹HNMR, which met the dissolution residue limit (< 0.5% wt).

### 6.6 Amplification preparation of gram grade of crystalline form III

Reducing the volume of DMSO, increasing the reaction temperature, lowering the temperature to room temperature after the crystallization and appropriately prolonging the slurrying time are beneficial to reducing the solvent residue and increasing the yield. Based on the above considerations, the amplification preparation of 3 g crystalline form III was based on a 1:4 DMSO/water system at 40°C, the amount of crystal seeds was 2% wt, and the drop rate of DMSO (5V) solution was controlled at 25 µL/minutes. To simulate production amplification, anchored agitator was used at a rotating speed of 150 rpm. The reaction system was suspended and slurried at 40 °C for 22 hours, and the XRPD results showed that the solid had been transformed to crystalline form III. In order to further improve the yield, the reaction temperature was lowered to room temperature, and the mixture was further suspended and slurried at room temperature, and after 22 hours, the solid sample was filtered and slurried in water to remove DMSO residue. As shown in Fig.42, 3 g of crystalline form III was successfully amplification prepared with a yield of 96.7%. As shown in Fig.43, 0.7% of the DMSO residue can be removed by slurring in water at room temperature and there is no transformation.

Considering the problems of compound production, solvent usage, dissolution residue and product yield, the crystallization process temperature of the final crystalline form III is 40 °C, and the reaction system is 1: 4 DMSO/ water, and the solvent volume is 25 V In summary, crystalline form III can be prepared by gram-scale amplification with a yield of up to 97%.

The XRPD of crystalline form III represented by 2θ angle is as shown in Fig.1. In TGA pattern of the crystalline form III, the crystalline form III has a weight loss of 1.5% in the range from room temperature to 100°C, and the "%" is the weight percentage. In DSC of the crystalline form III, the first endothermic peak is the removal of 0.4 water, and the second endothermic peak is attributed to the melt endothermic peak after sample dehydration, and the TGA and DSC pattern are as shown in Fig.2. After the crystalline form III is dehydrated, the crystalline form III quickly absorbs moisture under ambient humidity and becomes crystalline form III again, and the XRPD pattern before and after heating to dehydration are as shown in Fig.3. The dynamic moisture DVS of the crystalline form III shows that the sample has certain hygroscopicity, and the water content changes little in a wide humidity range, and the DVS pattern is as shown in Fig.4. In the XPRD pattern before and after the DVS test of the crystalline form III, there is no obvious change in the XRPD before and after the DVS test, and the XPRD pattern are as shown in Fig.5. In PLM pattern of the crystalline form III, the crystalline form is an irregular crystal of about 2 µm, and the agglomeration are 20 to 50 µm, and the PLM pattern is as shown in Fig.6.

### Embodiment 7: preparation and characterization of crystalline form IV of the compound of formula A

7.1 Crystalline form IV is obtained by moisture absorption of crystalline form I product under high humidity condition;
7.2 The slurry of crystalline form I product is slurried in 50 to 95% water/acetone (V/V) to obtain crystalline form IV

The XRPD of crystalline form IV represented by 2θ angle is substantially as shown in Fig.22. In TGA pattern of the crystalline form IV, there is a weight loss of 1.2% in the range of RT to 60°C. In DSC pattern of the crystalline form IV, there are two adjacent endothermic peaks, the first broad endothermic peak is presumed to be caused by dehydration, and the subsequent endothermic peak is a melting peak, and the TGA and DSC pattern are as shown in Fig.23. Combined with DSC and TGA pattern, it can be seen that the product is hydrate with about 0.34 water molecules. After the crystalline form IV is stable only in a high humidity environment, and after dehydration, the crystalline form IV quickly absorbs moisture under ambient humidity and becomes crystalline form I again, and the XRPD pattern before and after heating to dehydration are as shown in Fig.24. In PLM pattern of the crystalline form IV, the crystalline form is an irregular crystalline of about 5 µm, and the PLM pattern is as shown in Fig.25.

### Embodiment 8: preparation and characterization of crystalline form V of the compound of formula A

8.1 Solvent selection: water, the mixture was suspended and slurried at 50°C, and the concentration of slurring was 50 mg/mL, to obtain crystalline form V;
8.2 Solvent selection: ACN, the mixture was suspended and slurried at 50°C, the concentration of slurrying was 3.0 mg/mL, to obtain crystalline form V;
8.3 MeOH was selected as the solvent for volatile crystallization test to obtain crystalline form V

The XRPD of crystalline form V represented by 2θ angle is as shown in Fig.7. In TGA pattern of the crystalline form V, there is no weight loss in the temperature range of RT to 230°C. In DSC pattern of the crystalline form II, there is an endothermic peak at 166 °C ± 2 °C, and the melting enthalpy is 70±2J/g, and the TGA and DSC pattern are as shown in Fig.8. Combined with DSC and TGA pattern, it can be seen that the product is an anhydrous crystalline form. The DVS pattern of the crystalline form V shows that the sample has certain hygroscopicity (0.7%, 80%RH), and the DVS pattern is as shown in Fig.9. In the XPRD pattern before and after the DVS test of the crystalline form V, there is no obvious change in the XRPD before and after the DVS test, and the XPRD pattern are as shown in Fig.10. In PLM pattern of the crystalline form V, the crystalline form is an irregular crystalline of about 5 µm, and the PLM pattern is as shown in Fig.11.

### Embodiment 9: preparation and characterization of crystalline form VI of the compound of formula A

9.1 Crystalline form VI is obtained by slurrying crystalline form I or crystalline form V at 60 °C in a mixed solution in water/acetone with 10% water content (volume ratio) at 60 °C.

The X-ray powder diffraction pattern of crystalline form VI represented by 2θ angle is as shown in Fig.26. In the XRPD superimposition pattern of the crystalline form VI, the crystalline form sample transforms into crystalline form I after being placed under ambient humidity (35% RH) for a few minutes. The XRPD pattern is as shown in Fig.27. This indicates that crystalline form VI may be an extremely unstable hydrate.

### Embodiment 10: preparation and characterization of crystalline form VII of the compound of formula A

10.1 Solvent selection: ethylene glycol, the mixture was suspended and slurried at 90°C, the concentration of slurrying was 320 mg/mL, to obtain crystalline form VII;
10.2 The amorphous of compound A was slowly cooled and crystallized in ethylene glycol at the temperature of 50°C to RT to obtain crystalline form VII.

The XRPD of crystalline form VII represented by 2θ angle is substantially as shown in Fig.28. In the nuclear magnetic resonance ¹HNMR of crystalline form VII, the chemical shifts δ at 3.39 and 4.44 show residual ethylene glycol solvent, and the ¹HNMR pattern is as shown in Fig.29. In TGA pattern of the crystalline form VII, there is a weight loss of 25.7% in the range of RT to 120 °C. In DSC pattern of the crystalline form VII, there are two broad endothermic peaks, the first endothermic peak is presumed to be caused by desolventization, and the TGA and DSC pattern are as shown in Fig.30. Combined with DSC and TGA pattern, it can be seen that the product is a solvate containing 2.79 molecules of ethylene glycol.

### Embodiment 11: preparation and characterization of crystalline form VIII of the compound of formula A

11.1. Crystalline form III is used as seed crystal, at 40 °C, water was added to saturated 50% THF/ water saturated solution dropwise as anti-solvent, and the mixture was oily, and after cooling to room temperature, the mixture was continued slurrying to obtain crystalline form VIII (wet filter cake).

The XRPD of crystalline form VIII represented by 2θ angle is substantially as shown in Fig.31. In TGA pattern of the crystalline form VIII, there is a weight loss of 5.7% in the range of RT to 160°C. In the DSC pattern of the crystalline form VIII, there is only one endothermic peak, which is the melting peak after the sample is desolvated. Therefore, the crystalline form VIII is a solvate, and the TGA and DSC pattern are preferably as shown in Fig.32. In the nuclear magnetic resonance ¹HNMR of crystalline form VIII, the chemical shifts δ at 1.76 and 3.60 show residual THF solvent, and the ¹HNMR pattern is as shown in Fig.33. The crystalline form VIII contains 0.42 molecules of THF. The crystalline form VIII is unstable, and after dehydration (vacuum drying at 40°C for 3 hours), it is transformed into crystalline form I, and the XRPD pattern before and after heating are as shown in Fig.34.

### Embodiment 12: preparation and characterization of crystalline form IX of the compound of formula A

12.1 The crystalline form III was slurried in a 50% DMSO/water saturated solution at 40 °C to obtain crystalline form IX.

In TGA pattern of the crystalline form IX, the weight loss in the temperature range of RT-160°C is 18.23%, in the DSC pattern of the crystalline form IX, there is a corresponding endothermic peak corresponding to the TGA weight loss on the DSC pattern, and the TGA and DSC pattern are as shown in Fig.36. In the nuclear magnetic resonance ¹HNMR of crystalline form IX, the chemical shift δ at 2.68 shows residual DMSO solvent, and the ¹HNMR pattern is as shown in Fig.60. Combined with DSC and TGA pattern, it can be seen that the product is a DMSO solvate.

### Embodiment 13: Study on crystal transformation of crystalline forms

A total of nine different crystalline forms were obtained through screening experiments, including two amorphous forms (crystalline form I, crystalline form V), four solvates (crystalline form II, crystalline form VII, crystalline form IX, crystalline form VIII), and three hydrates (crystalline form III, crystalline form IV, crystalline form VI).

### 13.1 Crystal transformation between crystalline form I and crystalline form IV

The crystalline form I can be repeatedly obtained by different crystallization methods, but the crystalline form I showed hygroscopicity, and when the relative humidity was higher than 40%RH, the crystalline form I crystal was transformed into hydrate of crystalline form IV Therefore, crystalline form I has the risk of crystal transformation during API production and amplification.

### 13.2 Crystal transformation between other hydrate form and crystalline form I

Except crystalline form III, other hydrates are unstable and are transformed into crystalline form I after dehydration.
Crystalline form III (hemihydrate) has low risk of crystal transformation.
Crystalline form IV (hydrate) is transformed to crystalline form I after dehydration;
Crystalline form VI (hydrate) is extremely unstable, and it is quickly transformed to crystalline form I at ambient humidity.

### 13.3 Study on crystal transformation between amorphous form and hydrate

Water activity experiment was carried out. Crystalline forms I and V (both amorphous) were slurried in a mixed solvent in acetone/water with different water contents at room temperature and 60°C for three days, and after three days, solid sample was filtered to test XRPD, and the experimental results are as shown in Table 4. As shown in Fig.44 and 45, crystalline forms I and V are transformed into hydrate of crystalline form IV in acetone/water system with water content of 50-90% at room temperature or high temperature. When the water content is 10%, at high temperature, the crystalline form I or V is transformed into another hydrate (crystalline form VI). It is found that the hydrate is extremely unstable at ambient humidity (35% RH), and it is transformed into crystalline form I after standing for a few minutes. At room temperature, crystalline form V was transformed into crystalline form I in this solvent system.

**Table 4 Results of water activity experiments for crystalline forms I and V**

| Sample | Water content (%) | Reaction temperature (°C) | The experimental results | Remark |
|---|---|---|---|---|
| Crystalline form I | 10 | RT | Crystalline form I | N/A |
| | 50 | | Crystalline form IV | N/A |
| | 90 | | Crystalline form IV | N/A |
| | 10 | 60 | Crystalline form VI | Extremely unstable |
| | 50 | | Crystalline form IV | N/A |
| | 90 | | Crystalline form IV | N/A |
| Crystalline form V | 10 | RT | Crystalline form I | N/A |
| | 50 | | Crystalline form IV | N/A |
| | 90 | | Crystalline form IV | N/A |
| | 10 | 60 | Crystalline form VI | Extremely unstable |
| | 50 | | Crystalline form IV | N/A |
| | 90 | | Crystalline form IV | N/A |

| | | | | |
|---|---|---|---|---|
| N/A means: Not available | | | | |

### 13.4 Competition slurrying experiment between anhydrous crystalline forms

Competitive slurrying experiment was carried out: the same amount of crystalline forms I and V were weighed and mixed evenly, then XRPD was tested, and the results were used as T₀ of competitive slurrying experiment. The mixture was slurried in acetone, ethanol and water at room temperature and 60°C. Three days later, solid samples were obtained by filtration, and XRPD was tested. As shown in Fig.46, in organic solvent system (acetone or ethanol), crystalline form I can be obtained at room temperature or high temperature; crystalline forms I and V are competitive slurrying in water, and at room temperature, because the solubility is too low, and the crystalline form is still a mixed crystal, but at 60°C, the crystalline form is transformed into crystalline form V Combined with the water activity test results, it can be inferred that crystalline form I is more stable in organic solvent system or mixed solvent with low water content (≤10%), while crystalline form V is stable at high temperature (60°C) in water.

### 13.5 Crystal transformation between amorphous forms

After the same amount of crystalline form I and crystalline form V were lightly grinded and evenly mixed, the mixture was placed in an oven at 60°C and a room temperature dryer (to avoid crystalline form I moisture absorption) respectively, and after one week, XRPD was tested to study whether the crystalline forms were mutually transformed. As shown in Fig.47, after being placed under the above conditions for one week, crystalline form I and crystalline form V had no mutual crystal transformation trend, indicating that crystalline form I and crystalline form V are stable in solid state.

### Embodiment 14: Study on stability of crystalline forms

Compared with other crystalline forms, crystalline forms I, III and V show good solid properties, so their stability was tested respectively.

### 14.1 Study on Physical and Chemical Stability

About 10 mg of crystalline forms I, III and V were placed in the stability boxes at 40 °C/75 %RH (open) and 60°C (covered) for seven days, and some samples were taken out for XRPD and HPLC tests at 0 and 7 days, respectively, to investigate their physical and chemical stability. The results are as shown in Table 5, Fig 48,49 and 61, crystalline forms III and V did not change after seven days under the above test conditions, with no significant reduction in chemical purity. This indicates that crystalline form III and crystalline form V have good physical and chemical stability. As for crystalline form I, at 40°C/75%RH (open), the crystalline form absorbed moisture and was transformed into hydrate of crystalline form IV This result is consistent with that of DVS. After standing at 60°C for one week, the XRPD pattern of crystalline form I showed a change at 22-28 (2θ), and according to TGA results, there was a weight loss of 1.5% in the range from room temperature to 60°C, and then it was speculated that water absorption caused the change of diffraction peak. However, the crystalline form I has good chemical stability.

**Table 5 Evaluation results of stability**

| Sample | Initial purity (Peak area%) | Purity -7 days (peak area%) | | XRPD | |
|---|---|---|---|---|---|
| | | 40°C/75%RH | 60 °C | 40°C/75%RH | 60 °C |
| Crystalline form I (A10570-037) | 99.38 | 99.38 | 99.37 | Transformation to crystalline form IV | Similar to crystalline form I |
| Crystalline form V (A10570-031A1) | 99.29 | 99.28 | 99.30 | Constant | Constant |
| Crystalline form III (A10571-096B1) | 99.34 | 99.35 | 99.35 | Constant | Constant |

### 14.2 Mechanical stability test

The mechanical stability of crystalline forms I and III were investigated by grinding and pelleting. Grinding test method: the sample to be measured was put in a mortar and grinded for 2 minutes and 5 minutes respectively, then XRPD was test, and the changes of crystalline form and crystallinity were observed. Pelleting test method: the sample was placed in the die of tablet press, kept at 40 MPa for one minute, and then the crystalline form taken out to test XRPD.

The stability results of crystalline form I and crystalline form III after grinding (2 min and 5 min) and pelleting (40 MPa, 1 min) were as shown in Fig.50 and 51, and the crystallinity of crystalline form I decreased significantly after grinding, but the crystalline form remained unchanged. For crystalline form III, there is no obvious change in crystalline form and crystallinity after grinding (Fig.52), but the crystallinity decreases obviously after pelleting (Fig.53).

### Example 15: Solubility Study

Compared with other crystalline forms, crystalline forms I, III and V show good solid properties, so their solubility was tested respectively. The solubility of crystalline forms I, III and V in SGF, FaSSIF and FeSSIF respectively was tested, and the simulated body temperature was 37°C, and the test time was 24 hours.

10 mg of the sample was weighed, and then 2.0 mL of biological media buffer (i.e. the target concentration is 5 mg/mL) was added to form a suspension. The mixture was put in a constant temperature water bath shaker (37°C, 100 rpm), and sampled 500 mL at 0.5, 2 and 24 hours respectively. After filtration, the pH value and HPLC concentration of filtrate were tested respectively, and the solid sample obtained by filtration was tested by XRPD.

The solubility test results of crystalline forms I, III and V in biological media of SGF, FaSSIF and FeSSIF were as shown in Table 6 and Fig.54. The solubility of the above three crystalline forms in different biological media is similar, and the solubility in SGF is higher than 5 mg/mL. The solubility in FeSSIF is about 3 times that in FaSSIF, which indicates that food may help the absorption of drugs. During the test, the pH value of the biological media buffer has no obvious change.

As shown in Fig.55, crystalline form I is transformed into hydrate of crystalline form IV in FaSSIF and FeSSIF buffer for 0.5 hours. However, crystalline forms III and V did not change during the test, and the results are as shown in Fig.56 and Fig.57.

**Table 6 Solubility test results**

| Sample (batch number) | Mediu m | Solubility (mg/mL) | | | pH | | | | XRPD | | |
|---|---|---|---|---|---|---|---|---|---|---|---|
| | | 0.5 h | 2h | 24 h | 0 h | 0.5 h | 2h | 24 h | 0.5 h | 2h | 24 h |
| Crystalli ne form I (A1057 0-037) | SGF | Dissolution clarification | / | / | | 1.2 | 1.3 | 1. 2 | N/A | N/A | N/A |
| | FaSSI F | 0.026 | 0.025 | 0.034 | | 6.4 | 6.4 | 6. 4 | Crystalli ne form IV | Crystalli ne form IV | Crystalli ne form IV |
| | FeSSI F | 0.072 | 0.083 | 0.107 | | 5.0 | 5.1 | 5. 1 | Crystalli ne form IV | Crystalli ne form IV | Crystalli ne form IV |
| Crystalli ne form V (A1057 0-031) | SGF | 4.331 | Dissolution clarification | / | | 1.2 | 1.1 | 1. 2 | N/A | N/A | N/A |
| | FaSSI F | 0.030 | 0.034 | 0.045 | | 6.4 | 6.4 | 6. 4 | Unchang ed | Unchang ed | Unchang ed |
| | FeSSI F | 0.091 | 0.093 | 0.121 | | 5.0 | 5.0 | 5. 1 | Unchang ed | Unchang ed | Unchang ed |
| Crystalli ne form III (A1057 0-096) | SGF | Dissolution clarification | / | / | | 1.0 | / | / | N/A | N/A | N/A |
| | FaSSI F | 0.023 | 0.025 | 0.033 | | 6.4 | 6.4 | 6. 4 | Unchang ed | Unchang ed | Unchang ed |
| | FeSSI F | 0.074 | 0.088 | 0.120 | | 4.9 | 4.8 | 4. 9 | Unchang ed | Unchang ed | Unchang ed |

| | | | | | | | | | | | |
|---|---|---|---|---|---|---|---|---|---|---|---|
| "Dissolution clarification": indicates that the solubility of the sample in the medium is greater than 5 mg/mL by visual observation; "N/A": indicates no solid sample to test the XRPD. | | | | | | | | | | | |

### Embodiment 16: Pharmacokinetic Study

Beagle dog (general grade, Beijing Masi Biotechnology Co., Ltd.) was given compound A by single oral gavage to study the pharmacokinetics of the compound in plasma. The test drug (100 mg/kg dosage, 5 mL/kg administration volume) was given by gavage, and samples were collected at 10 minutes, 30 minutes, 1 hour, 2 hours, 4 hours, 6 hours, 8 hours, 10 hours, 24 hours and 48 hours after administration: peripheral venipuncture was performed, and about 1.0 mL whole blood was collected from each animal at every time point, and blood samples were collected. Within 60 minutes after blood collection, 0.2 mL of whole blood was separated into a cryopreservation tube, and deionized water was added at a ratio of 1:1, which was mixed upside down and then frozen. The remaining samples were centrifuged at 2,000g at 2 to 8 °C for 10min to obtain plasma samples, and the hemolysis of each sample was recorded, and the influence on detection was evaluated, and then the color of plasma was observed and recorded on the blood collection table ("-"means no hemolysis, "+" means pink supernatant and "++" means red supernatant).

The concentration of compound A in plasma was determined by LC-MS/MS method (Non-GLP). The pharmacokinetic parameters were calculated by WinNonlin (PhoenixTM, version 8.1) or other similar software. If applicable plasma drug concentration-time data are available, the following pharmacokinetic parameters are calculated: CL (clearance), Vₛₛ (steady-state apparent volume of distribution), T_{1/2} (elimination half-life), Cₘₐₓ (peak concentration), Tₘₐₓ (peak time), AUC (area under the concentration-time curve), MRT (mean residence time). Pharmacokinetic data were described by descriptive statistics, such as mean, standard deviation and sample size. Calculations were performed with either Microsoft Excel 2007 or 2010.

### 16.1 Comparison of PK properties between crystalline form I and crystalline form V

Comparison of crystalline form I and crystalline form V is the same for 3 dogs (male), 10mg/kg po, fasting, 0.5%CMC-Na stirring for 3 hours, and then administration (suspension), and the data obtained are as shown in Table 7.

**Table 7 PK comparison between crystalline form I and crystalline form V**

| Group | Cₘₐₓ (ng/mL) | Tₘₐₓ (hr) | T_{1/2} (hr) | AUCₗₐₛₜ (ng/mL*hr) | AUC_{INF} (ng/mL *hr) | MRTₗₐₛₜ(hr) | F(%) |
|---|---|---|---|---|---|---|---|
| Crystalline form I | 991.33 | 2.00 | 11.91 | 7926.33 | 8937.13 | 8.11 | 35.2% |
| Crystalline form V | 170.43 | 4.67 | 44.35 | 2637.43 | 14928.54 | 10.39 | 11.7% |

Calculating AUC (crystalline form I): AUC (crystalline form V) = 300.53%, it was found that the exposure level and absolute bioavailability of crystalline form V were less than those of crystalline form I.

### 16.2 Pharmacokinetics study in crystalline form III

Comparing the female difference of oral administration of crystalline form III & fasting or not, 100mg/kg, po, fasting & non-fasting, 0.5%CMC-Na after stirring for 8 hours, and suspending, the data obtained are as shown in Table 8.

**Table 8 Pharmacokinetics data of crystalline form III**

| Group | Sample | Gen der | Cₘₐₓ (ng/mL) | Tₘₐₓ (hr) | T_{1/2} (hr) | AUCₗₐₛₜ (ng/mL*hr) | AUC_{INF} (ng/mL*hr) | AUC ₀₋₂₄ₕ (ng/mL*hr) | MRTₗₐₛₜ (hr) |
|---|---|---|---|---|---|---|---|---|---|
| Crystal line form III 100mg /kg (po,fas t) | plasma | male | 5696.67 | 9.00 | N/A | 81386.00 | 40212.50 | 48827.00 | 13.62 |
| | plasma | fema le | 1683.00 | 1.33 | 12.00 | 8162.67 | 9205.33 | 7542.33 | 8.06 |
| | blood | male | 14633.33 | 9.33 | 14.88 | 449055.58 | 521498.85 | 254535.58 | 19.48 |
| | blood | fema le | 10266.67 | 1.83 | 22.38 | 186670.70 | 241633.98 | 133666.70 | 14.07 |
| Crystal line form III 100mg /kg (po,No n-fast) | plasma | male | 18866.67 | 4.67 | 5.33 | 365963.00 | 366780.33 | 304669.33 | 11.83 |
| | plasma | fema le | 24000.00 | 7.33 | 5.14 | 465025.67 | 465964.00 | 377730.67 | 12.63 |
| | blood | male | 19000.00 | 4.67 | 28.57 | 557471.56 | 817608.93 | 352991.56 | 18.85 |
| | blood | fema le | 23466.67 | 6.00 | 25.13 | 675026.56 | 954919.17 | 431706.56 | 18.69 |

| | | | | | | | | | |
|---|---|---|---|---|---|---|---|---|---|
| N/A: not available | | | | | | | | | |

The results showed that under fasting condition, there were some differences in plasma and whole blood drug concentrations between male and female dogs, the male in plasma was about 6 times higher than that of male and female, and the male in whole blood was 1.9 times higher than that of male and female. However, under the condition of non-fasting, there was no significant difference in plasma and whole blood drug concentrations between male and female dogs, and under fasting condition, the distribution of red blood cells in male and female dogs was different (male and female blood: plasma = 1452% vs 521%), but under non-fasting condition, there was no significant difference (male and female blood: plasma = 114% vs 116%), and the drug concentration in plasma and whole blood under non-fasting condition was higher than that under non-fasting condition for both males and females, and then the rising level of plasma drug concentration under non-fasting condition is higher than that of whole blood, and the female dog's plasma drug concentration is most affected by fasting or not. The increase of whole blood drug concentration may be due to the increase of drug absorption under the condition of non-fasting, which leads to the increase of the overall exposure level *in vivo,* and it is speculated that diet is conducive to compound A absorption; however, the increase of plasma drug concentration is higher than that of whole blood, which may be due to:
1. the drug concentration in red blood cells is saturated, which leads to a similar sudden increase in plasma drug concentration (presumably the highest possibility);
2. after the first day of fasting administration, the drugs continued to accumulate in red blood cells, and the 7-day cleaning period was not enough to completely metabolize the drugs distributed in red blood cells, so the second time of non-fasting administration led to a significant increase in plasma drug concentration;
3. after fasting administration on the first day, it affected red blood cells, which reduced the ability of combining red blood cells with drugs during the second non-fasting administration, resulting in a significant increase in plasma drug concentration.

### Biological test

### Example A In vitro biological activity evaluation

The antagonistic properties of the compounds of the present disclosure were determined by the FLIPR (fluorescence imaging plate reader) method, and the compounds were inhibitors of intracellular calcium elevation induced by activation of hP2X3 (human purinergic P2X receptor subtype 3, the login number NM_002559.4) expressed in HEK293 cells (human renal epithelial cell line, ATCC).

HEK293 cells stably expressing hP2X3 were placed and cultured in a cell incubator with a humidity of 5% at 37°C in a DMEM high-glucose medium containing 10% FBS (fetal bovine serum, Gibco, 10099-141), 1% penicillin-streptomycin (Gibco, 15140-122), and 1 mg/mL G418 (Invitrogen, 10131027). 18-24 hours before the FLIPR experiment, cells were seeded into 384 wells (10,000 cells/well) at a density of 400,000 cells/mL and incubated overnight in a cell culture incubator. On the day of the experiment, the culture medium was discarded, and the cells were washed with FLIPR buffer (each 30 mL buffer containing 0.3 mL probenecid (Thermo, P36400), 0.6 mL of 1M HEPES (Invitrogen, 15630080) and 29.1mL HBSS(Invitrogen, 14065056)). 20 µL of 0.5×Calcium 6 fluorescent dye (Molecular Devices, R8190) was added per well and incubated with dye loading for 1.5 h at 37 °C. 10 µL of test compound (dissolved at 10 mM in DMSO and serially diluted with buffer) or vehicle was then added to each well and allowed to equilibrate for 30 min at room temperature. The cell plate was then placed in the FLIPR for baseline fluorescence measurements (excitation at 485 nm and emission at 525-535 nm). Then agonist (BZ-ATP (Sigma, B6396) at a final concentration of 2.5 µM) or vehicle (ultrapure water) was added at 10 µL/well, the fluorescence value was measured at 1-second intervals for 2 minutes, and finally the output fluorescence was counted analysis.

The IC₅₀ obtained using the above method was as shown in the following Table.

**Table 9 The IC₅₀ using the above method.**

| Compound | P2X3 IC₅₀ (µM) |
|---|---|
| Compound 172 | 0.0007 |
| Positive compound 1 | 0.0115 |
| Positive compound 2 | 0.0522 |

Positive compound 1:

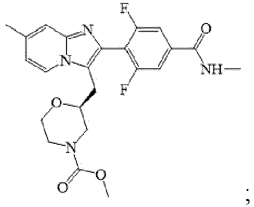

Positive compound 2:

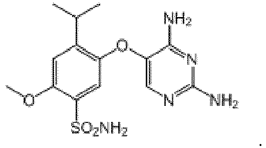

### Embodiment B In vitro P2X2/3 receptor selective evaluation

The selectivity of the compound of the present disclosure to P2X2/3 receptor was determined by the FLIPR (fluorescence imaging plate reader) method, and the compound was an inhibitor of intracellular calcium elevation induced by activation of hP2X2/3 (heterodimeric receptors formed by human purinergic P2X receptor subtypes 2 and 3, the login number of P2X2 is NM_002559.4, the login number of P2X3 is NM_002559.4) expressed in HEK293 cells (human renal epithelial cell line, ATCC).

HEK293 cells stably expressing hP2X2/3 were placed and cultured in a cell incubator with a humidity of 5% at 37°C in a DMEM high-glucose medium containing 10% FBS (fetal bovine serum, Gibco, 10099-141), 1% penicillin-streptomycin (Gibco, 15140-122), and 1 mg/mL G418 (Invitrogen, 10131027). 18-24 hours before the FLIPR experiment, cells were seeded into 96 wells (25000 cells/well) at a density of 250000 cells/mL and incubated overnight in a cell culture incubator. On the day of the experiment, the culture medium was discarded, and the cells were washed with FLIPR buffer (each 30 mL buffer containing 0.3 mL of probenecid (Thermo, P36400), 0.6 ml of 1M HEPES (Invitrogen, 15630080) and 29.1 mL HBSS(Invitrogen, 14065056)). 75 µL 1 mM Fluo-4 AM fluorescent dye (Thermo, F14202) was added to each well, and the dye load was incubated for 1.0 hour at 37°C. Then, the 96-well plate was washed once with buffer, and 50 µL of buffer containing test compound or vehicle was added to each well, and it was incubated at room temperature for 30 minutes. The cell plate was then placed in the FLIPR for baseline fluorescence measurements (excitation at 485 nm and emission at 525-535 nm). Then agonist (BZ-ATP (Sigma, B6396) at a final concentration of 5 µM) or vehicle (ultrapure water) was added at 50 µL/well, the fluorescence value was measured at 1-second intervals for 2 minutes, and finally the output fluorescence was counted analysis.

The IC₅₀ obtained using the above method was as shown in the following Table.

**Table 10 The IC₅₀ using the above method.**

| Compound | IC₅₀ | Selectivity multiple |
|---|---|---|
| Compound 172 | >60 | >85714 |
| Positive compound 1 | >60 | >5217 |
| Positive compound 2 | 0.36 | 6.9 |

### Embodiment C Activity test of simple citric acid cough model

Male Dunkin Hartley guinea pigs (300-350 g) were placed in the animal atomization box, the door of the atomization box was closed, and the ultrasonic atomizer (Guangdong Yuehua) was opened at the same time, at the maximum atomization amount (about 2 mL/min) 17.5% citric acid gas was filled with the atomization box, which was continuously atomized for 20 seconds, and the cough performance of the animals was continuously observed within 10 min after the atomization started. During the 10-minute observation period, manual counting of coughs was required, and the number of coughs was judged according to the guinea pig's coughing posture, such as abdominal twitching, mouth opening, head down hooking, etc., as well as the sound of coughing, the number of coughs in the first 5 minutes and the number of coughs in the first 10 minutes were recorded, and the cough incubation period of guinea pigs was also recorded, that is, the time from citric acid induction to the first cough as well as the sound of coughing.

**Table 11 Animal experiments**

| Group | | Average cough times | Cough suppression rate Vs Vehicle (%) | Cough suppression rate Vs Base value (%) | Average cough latency |
|---|---|---|---|---|---|
| Vehicle group | Before administration | 17.22 | / | / | 108.44 |
| | After administration | 18.33 | | | 105.78 |
| Positive compoun d 1 30 mg/kg | Before administration | 18.63 | 42.6 | 47.0 | 80.13 |
| | After administration | 9.88 | | | 226.75 |
| Compou nd 172 30 mg/kg | Before administration | 19.56 | 43.9 | 50.6 | 87.56 |
| | After administration | 9.67 | | | 118.89 |

Compound 172 has the effect of reducing the number of coughs and increasing the incubation period of coughs, and is comparable to the efficacy of positive compound.

### Embodiment D Activity test of ATP-citric acid cough model

Male Dunkin Hartley guinea pigs (300-400 g) were placed in a whole-body volume scanning box for 3 to 5minutes, and then subjected to ATP atomization for 2 minutes, with an interval of 3 minutes, followed by citric acid atomization for 5 minutes, and all atomization rates were about 300 µL/min. From citrate acid atomization, cough times and cough latency of animals within 10 min were recorded. During the 10-minute observation period, manual counting of coughs was required, and the number of coughs was judged according to the guinea pig's coughing posture, such as abdominal twitching, mouth opening, head down hooking, etc., as well as the sound of coughing, the number of coughs in the first 10 minutes were recorded, and the cough incubation period of guinea pigs was also recorded.

Animals were divided into vehicle group, dextromethorphan (63mg/kg) positive group, AF-219(30mg/kg) positive group and compound 172 (3,10,30mg/kg) administration group of Embodiment. All compounds were administered orally, except dextromethorphan, which was administered 40 minutes before ATP-citric acid exposure, and the rest compounds were ATP- citric acid.

**Table 12 Quinine/water consumption ratio obtained for compound**

| Compound | | Coughing times in 10min | Cough suppression rate vs Vehicle (%) |
|---|---|---|---|
| Vehicle | | 20.83 | |
| Dextromethorphan group 63mg/kg | | 8.00 | 61.60 |
| AF-219 | 30 mg/kg | 11.42 | 45.20 |
| Compound 172 | 3 mg/kg | 14.42 | 30.80 |
| | 10 mg/kg | 10.17 | 51.20 |
| | 30 mg/kg | 9.83 | 52.80 |

As shown in Table 5, after treatment, all compounds have tendency to decrease the number of coughs compared with the vehicle group, in which both dextromethorphan positive group and compound 172 can significantly reduce the number of coughs, and compound 172 has a dose-dependent inhibitory effect. Compared with the positive compound 2, compound 172 has stronger cough inhibitory effect.

### Embodiment E Cytotoxicity test in vitro

In vitro cytotoxicity test of the compounds in the present disclosure was carried out in HepG2 cells by CCK-8 method. HepG2 cells in logarithmic phase was collected, the concentration of cell suspension was adjusted, a 96-well cell culture plate with 50000 cells/well was plated, and the cell was incubated overnight in a 5%, 37°C cell incubator, after the cell fusion degree in the plate reached 80-90%, solution was changed and added test compounds or vehicle (DMSO) with different concentration gradients, and incubated for 48 hours in a 5%, 37°C cell incubator. After the treatment, the medium in the plate was discarded, washed twice with PBS, 100 µL of CCK-8 working solution (Biyuntian Biotechnology) was added to each well, and incubated at 37 °C for 1.5 h in the dark and the absorbance values of the wells at OD_{450 nm} were analyzed to calculate the CC₅₀ of each compound.

**Table 13 IC₅₀ value of each compound**

| Compound | HepG2 CC₅₀ (µM) |
|---|---|
| Compound 172 | 57.05 |
| Positive compound 1 | 128.4 |
| Positive compound 2 | >200 |

### Embodiment F Metabolic stability test in vitro

The in vitro metabolic stability of the compounds of the present disclosure was determined using the body temperature incubation method of various hepatic microparticles. In the liver microsomal reaction system (1 mg/mL liver microsomal protein, 25 U/mL glucose 6-phosphate dehydrogenase, 1 mM NADP, 6 mM D-6- phosphate glucose, 5 mM MgCh), a proper amount of test compounds was added, and the reaction solution was started by incubation in a water bath at 37 °C, at each time point, 100 µL of the reaction solution was added into a centrifuge tube containing 400 µL of internal standard working solution precooled at 0°C (acetonitrile solution containing 200 ng/mL dexamethasone, diclofenac, tolbutamide and labetalol), reaction was stopped, centrifuged at 4°C for 10 min, and the supernatant was taken and analyzed by LC-MS to obtain the in vitro metabolic half-life of the tested compounds in various liver microsomes.

**Table 14 In vitro metabolic stability test**

| Compound | Human liver microsomes T_{1/2} min | Rat liver microsomes T_{1/2} min | Guinea pig liver microsomes T_{1/2} min |
|---|---|---|---|
| Embodiment 172 | 824.3 | 223.4 | 410.3 |
| Positive compound 1 | 1454.06 | 53.42 | 112.04 |
| Positive compound 2 | NA | 1090.01 | 892.21 |

### Embodiment G Taste Disorder Test by Double Bottle Method

SPF male SD rats (6-8 weeks) received 3 days of adaptive drinking water training immediately after storage, and the specific training content was that animals were kept in a single cage, and two bottles of water (both common drinking water) were placed in each cage, and during the adaptive training, water treatment was forbidden all night (the drinking water bottle was removed), and drinking water was given again at 8: 30 am to 5: 30 pm, and then this cycle lasted for 3 days, and the two bottles of water were changed every day, and during the whole adaptive training period, animals were ate freely. All animal water bottles were removed 20 hours before the formal experiment, and water was banned until the time of the experiment. During the formal experiment, all animals were randomly divided into groups, and the test compound or vehicle was given before the water supply was renewed, and the drug was given by single intraperitoneal injection, and the administration time was determined according to the Tₘₐₓ of the test compound. Then, the animals were placed in a single cage, and given two bottles of drinking water, one bottle of ordinary drinking water and one bottle of 0. 3 mM tonic water, and the water consumption of animals was observed within 15 minutes, and the quinine water consumption/ordinary water consumption was statistically analyzed, with positive compound 2 as the positive control of taste disorder.

**Table 15 Animal drinking water quantity test**

| Compound | | Quinine/tap water * 100% |
|---|---|---|
| Vehicle | | 7.19% |
| Positive compound 2 | 10 | 18.62% |
| | 20 | 34.99% |
| Compound 172 | 5 | 7.05% |
| | 10 | 7.41% |
| | 20 | 8.66% |

The results showed that compared with the blank vehicle group, compound 172 of 5, 10 and 20mg/kg had no obvious effect on the quinine/water ratio of rats, indicating that compound 172 had little effect on animal taste, and compared with the positive compound 2, the proportion of quinine/water consumption is significantly lower than that of the positive compound 2, which indicates that the risk of taste disorder caused by the compound 172 is far better than that of the positive compound 2, and the safety is better.

## Claims

1. A crystalline form of the compound represented by formula A or a solvate thereof: 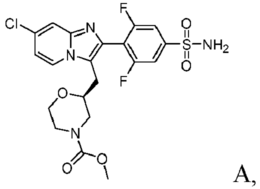
the crystalline form is selected from crystalline form I, crystalline form II, crystalline form III, crystalline form IV, crystalline form V, crystalline form VI, crystalline form VII crystalline form VIII and crystalline form IX;
wherein, the crystalline form III of hydrate of the compound of formula A has an X-ray powder diffraction pattern comprising characteristic diffraction peaks at the following angles of 2θ: 12.91°± 0.20° 16.77°± 0.20°, 19.27° ± 0.20° and 22.80°±0.20°;
the crystalline form V of the compound of formula A has an X-ray powder diffraction pattern comprising characteristic diffraction peaks at the following angles of 2θ: 8.38°± 0.20°, 9.15° ± 0.20°, 13.52° ± 0.20° and 18.44 ± 0.20°;
the crystalline form I of the compound of formula A has an X-ray powder diffraction pattern comprising characteristic diffraction peaks at the following angles of 2θ: 8.56 °± 0.20°, 12.48 ± 0.20° and 22.13° ± 0.20°;
the crystalline form II of MTBE solvate of the compound of formula A has an X-ray powder diffraction pattern comprising characteristic diffraction peaks at the following angles of 2θ: 8.42°±0.20°, 12.09° ± 0.20°, 13.68° ± 0.20° and 20.87° ± 0.20°;
the crystalline form IV of hydrate of the compound of formula A has an X-ray powder diffraction pattern comprising characteristic diffraction peaks at the following angles of 2θ: 8.65° ± 0.20°, 12.69° ± 0.20° and 22.56° ± 0.20°;
the crystalline form VI of hydrate of the compound of formula A has an X-ray powder diffraction pattern comprising characteristic diffraction peaks at the following angles of 2θ: 8.62° ± 0.20°, 12.69° ± 0.20° and 22.59° ± 0.20°;
the crystalline form VII of ethylene glycol solvate of the compound of formula A has an X-ray powder diffraction pattern comprising characteristic diffraction peaks at the following angles of 2θ: 8.36°± 0.20°, 12.13 °± 0.20°, 12.45° ± 0.20°, 16.84° ± 0.20° and 21.66° ± 0.20°;
the crystalline form VIII of THF solvate of the compound of formula A has an X-ray powder diffraction pattern comprising characteristic diffraction peaks at the following angles of 2θ: 8.53°±0.20°, 12.38° ± 0.20°, 13.66° ± 0.20° and 21.49° ± 0.20°;
the crystalline form IX of DMSO solvate of the compound of formula A has an X-ray powder diffraction pattern comprising characteristic diffraction peaks at the following angles of 2θ: 8.55°±0.20°, 12.43° ± 0.20°, 21.75° ± 0.20° and 25.07° ± 0.20°.

2. The crystalline form of compound A or the solvate thereof according to claim 1, wherein,
the crystalline form III has an X-ray powder diffraction comprising characteristic diffraction peaks at the following angles of 2θ: 12.91°±0.20°, 16.77°±0.20°, 19.27°± 0.20°, 22.80 °± 0.20°, 13.75° ± 0.20°, 14.46° ± 0.20° and 20.86° ± 0.20°;
and/or, the crystalline form V has an X-ray powder diffraction pattern comprising characteristic diffraction peaks at the following angles of 2θ: 8.38 °± 0.20°, 9.15° ± 0.20°, 13.52° ± 0.20° 18.44±0.20°, 16.26°±0.20°, 16.89°±0.20° and 17.86° ± 0.20°;
and/or, the crystalline form I has an X-ray powder diffraction pattern comprising characteristic diffraction peaks at the following angles of 2θ: 8.56°± 0.20°, 12.48°± 0.20°, 22.13 °± 0.20°, 13.53°± 0.20°, 14.25°± 0.20°, 25.18°± 0.20° and 26.07° ± 0.20°;
and/or, the crystalline form II has an X-ray powder diffraction pattern comprising characteristic diffraction peaks at the following angles of 2θ: 8.42°± 0.20°, 12.09°±0.20°, 13.68°±0.20°, 20.87°± 0.20°, 16.17 °± 0.20°, 16.93° ± 0.20°, 17.55° ± 0.20° and 21.20° ± 0.20°;
and/or, the crystalline form IV has an X-ray powder diffraction pattern comprising characteristic diffraction peaks at the following angles of 2θ: 8.65°±0.20°, 12.69°±0.20°, 22.56°± 0.20°, 13.48 °± 0.20°, 17.39° ± 0.20°, 21.04° ± 0.20° and 23.63° ± 0.20°;
and/or, the crystalline form VI has an X-ray powder diffraction pattern comprising characteristic diffraction peaks at the following angles of 2θ: 8.62°± 0.20°, 12.69°±0.20°, 22.59°±0.20°, 13.46°± 0.20°, 17.41 °± 0.20°, 26.51° ± 0.20°, 25.62° ± 0.20° and 25.24° ± 0.20°;
and/or, the crystalline form VII has an X-ray powder diffraction pattern comprising characteristic diffraction peaks at the following angles of 2θ: 8.36°±0.20°, 12.13°±0.20°, 12.45°± 0.20°, 16.84 °± 0.20°, 21.66° ± 0.20°, 21.07° ± 0.20° and 24.82° ± 0.20°;
and/or, the crystalline form VIII has an X-ray powder diffraction pattern comprising characteristic diffraction peaks at the following angles of 2θ: 8.53°±0.20°, 12.38°±0.20°, 13.66°± 0.20°, 21.49 °± 0.20°, 20.99° ± 0.20°, 24.94° ± 0.20° and 25.31° ± 0.20°;
and/or, the crystalline form IX has an X-ray powder diffraction pattern comprising characteristic diffraction peaks at the following angles of 2θ: 8.55°± 0.20°, 12.43°±0.20°, 21.75°±0.20°, 25.07°± 0.20°, 13.57 °± 0.20°, 17.18° ± 0.20°, 20.94° ± 0.20° and 25.57° ± 0.20°.

3. The crystalline form of compound A or the solvate thereof according to claim 2, wherein,
the crystalline form III has an X-ray powder diffraction pattern comprising characteristic diffraction peaks at the following angles of 2θ: 12.91°±0.20°, 16.77±0.20°, 19.27°± 0.20°, 22.80°±0.20°, 13.75°±0.20°, 14.46°± 0.20°, 20.86 °± 0.20°, 21.08° ± 0.20°, 23.75° ± 0.20° and 24.05° ± 0.20°;
and/or, in thermogravimetric analysis pattern of the crystalline form III, the crystalline form **III** has a weight loss of 1.5% in the range from room temperature to 100°C;
and/or, in differential scanning calorimetric pattern of the crystalline form III, the first endothermic peak is the removal of 0.4 water;
and/or, the crystalline form V has an X-ray powder diffraction pattern comprising characteristic diffraction peaks at the following angles of 2θ: 8.38°±0.20°, 9.15°±0.20°, 13.52°± 0.20°, 18.44°±0.20°, 16.26°±0.20°, 16.89°± 0.20°, 17.86 °± 0.20°, 22.35° ± 0.20°, 23.56° ± 0.20° and 24.74° ± 0.20°;
and/or, in differential scanning calorimetry pattern of the crystalline form I, there is an endothermic peak at 166 °C, and the melting enthalpy is 70±2J/g;
and/or, the crystalline form I has an X-ray powder diffraction pattern comprising characteristic diffraction peaks at the following angles of 2θ: 8.56°±0.20°, 12.48±0.20°, 22.13°± 0.20°, 13.53°±0.20°, 14.25±0.20°, 25.18°± 0.20°, 26.07 °± 0.20°, 22.32° ± 0.20°, 23.23° ± 0.20° and 23.42° ± 0.20°;
and/or, in differential scanning calorimetry pattern of the crystalline form I, there is an endothermic peak at 152 °C, and the melting enthalpy is 44±2J/g;
and/or, the crystalline form II has an X-ray powder diffraction pattern comprising characteristic diffraction peaks at the following angles of 2θ: 8.42°±0.20°, 12.09°±0.20°, 13.68°±0.20°, 20.87°± 0.20°, 16.17°±0.20°, 16.93°±0.20°, 17.55°± 0.20°, 21.20 °± 0.20°, 22.60° ± 0.20°, 23.23° ± 0.20° and 24.40° ± 0.20°;
and/or, in thermogravimetric analysis pattern of crystalline form II, there is a weight loss of 3.5% in the temperature range of 100 to 160 °C and a weight loss of 2.9% in the range of 160 to 200 °C;
and/or, the crystalline form IV has an X-ray powder diffraction pattern comprising characteristic diffraction peaks at the following angles of 2θ: 8.65°±0.20°, 12.69°±0.20°, 22.56°± 0.20°, 13.48°±0.20°, 17.39°±0.20°, 21.04°± 0.20°, 23.63 °± 0.20°, 14.39° ± 0.20°, 25.60° ± 0.20° and 26.52° ± 0.20°;
and/or, in TGA pattern of the crystalline form IV, there is a weight loss of 1.2% in the range of RT to 60°C;
and/or, the crystalline form VII has an X-ray powder diffraction pattern comprising characteristic diffraction peaks at the following angles of 2θ: 8.36°±0.20°, 12.13°±0.20°, 12.45°± 0.20°, 16.84°±0.20°, 21.66°±0.20°, 21.07°± 0.20°, 24.82 °± 0.20°, 13.61° ± 0.20°, 23.22° ± 0.20° and 24.57° ± 0.20°;
and/or, in TGA pattern of the crystalline form VII, there is a weight loss of 25.7% in the range of room temperature to 120 °C;
and/or, the crystalline form VIII has an X-ray powder diffraction pattern comprising characteristic diffraction peaks at the following angles of 2θ: 8.53°±0.20°, 12.38°±0.20°, 13.66°± 0.20°, 21.49°±0.20°, 20.99°±0.20°, 24.94°± 0.20°, 25.31 °± 0.20°, 17.14° ± 0.20°, 21.72° ± 0.20° and 23.00° ± 0.20°;
and/or, in thermogravimetric analysis pattern of the crystalline form VIII, there is a weight loss of 5.7% in the range of room temperature to 160°C;
and/or, the crystalline form IX has an X-ray powder diffraction pattern comprising characteristic diffraction peaks at the following angles of 2θ: 8.55°±0.20°, 12.43°±0.20°, 21.75°± 0.20°, 25.07°±0.20°, 13.57°±0.20°, 17.18°± 0.20°, 20.94 °± 0.20°, 25.57° ± 0.20°, 21.37° ± 0.20° and 23.12° ± 0.20°;
and/or, in TGA pattern of the crystalline form IX, there is a weight loss of 18.23% in the range of room temperature to 60°C.

4. The crystalline form of compound A or the solvate thereof according to claim 3, wherein,
the crystalline form III has an X-ray powder diffraction pattern represented by 2θ angle substantially as shown in Fig. 1;
and/or, the crystalline form III has a thermogravimetric analysis pattern and a differential scanning calorimetric pattern substantially as shown in Fig.2;
and/or, the crystalline form III has a dynamic moisture adsorption pattern as shown in Fig.4;
and/or, the crystalline form III has a polarizing microscope pattern substantially as shown in Fig.6;
and/or, the crystalline form III contains 0.4 equivalent of water;
and/or, the crystalline form V has an X-ray powder diffraction pattern represented by 2θangle substantially as shown in Fig.7;
and/or, the crystalline form V has a dynamic moisture adsorption pattern as shown in Fig.9;
and/or, the crystalline form V has a thermogravimetric analysis pattern and a differential scanning calorimetric pattern substantially as shown in Fig.8;
and/or, the crystalline form V has a polarizing microscope pattern substantially as shown in Fig. 11;
and/or, the crystalline form I has an X-ray powder diffraction pattern represented by 2θ angle substantially as shown in Fig. 12;
and/or, the crystalline form I has a thermogravimetric analysis pattern and a differential scanning calorimetric pattern substantially as shown in Fig. 13;
and/or, the crystalline form I has a dynamic moisture adsorption pattern as shown in Fig. 14;
and/or, the crystalline form I has the polarizing microscope pattern substantially as shown in Fig. 17;
and/or, the crystalline form II has a nuclear magnetic resonance pattern as shown in Fig. 19;
and/or, the crystalline form II has an X-ray powder diffraction pattern represented by 2θ angle substantially as shown in Fig. 18;
and/or, the crystalline form II has the polarizing microscope pattern substantially as shown in Fig.21;
and/or, the crystalline form II has a thermogravimetric analysis pattern and a differential scanning calorimetric pattern substantially as shown in Fig.20;
and/or, the crystalline form IV has an X-ray powder diffraction pattern represented by 2θ angle substantially as shown in Fig.22;
and/or, the crystalline form IV has a thermogravimetric analysis pattern and a differential scanning calorimetric pattern as shown in Fig.23;
and/or, the crystalline form IV has a polarizing microscope pattern substantially as shown in Fig.25;
and/or, the crystalline form VI has an X-ray powder diffraction pattern represented by 2θ angle substantially as shown in Fig.26;
and/or, the crystalline form VII has an X-ray powder diffraction pattern represented by 2θ angle substantially as shown in Fig.28;
and/or, the crystalline form VII has a nuclear magnetic resonance pattern as shown in Fig.29;
and/or, the crystalline form VII has a thermogravimetric analysis pattern and a differential scanning calorimetric pattern substantially as shown in Fig.30;
and/or, the crystalline form VIII has an X-ray powder diffraction pattern represented by 2θ angle substantially as shown in Fig.31;
and/or, the crystalline form VIII has a thermogravimetric analysis pattern and a differential scanning calorimetric pattern substantially as shown in Fig.32;
and/or, the crystalline form VIII has a nuclear magnetic resonance pattern as shown in Fig.33;
and/or, the crystalline form IX has an X-ray powder diffraction pattern represented by 2θ angle substantially as shown as in Fig.35;
and/or, the crystalline form IX has a thermogravimetric analysis pattern and a differential scanning calorimetric pattern substantially as shown in Fig.36.

5. A preparation method of the crystalline form III of substance A, wherein, the preparation method is scheme 1, scheme 2 or scheme 3; wherein,
scheme 1 comprises the following steps: transforming the suspension of the amorphous of the compound of formula A and solvent to a crystalline form III of substance A; the solvent is water or alcohol solvent;
scheme 2 comprises the following steps: adding anti-solvent into the solution of the compound of formula A and a solvent, and crystallizing into a crystalline form III of by substance A; the solvent is selected from the group consisting of alcohols, furans and DMSO; and the anti-solvent is water;
scheme 3 comprises the following steps: adding a solution of compound A and a solvent into aqueous solution A, and crystallizing into a crystalline form III of substance A, wherein the aqueous solution A is the suspension of crystal seeds of crystalline form III of substance A and water; and the solvent is DMSO.

6. The preparation method according to claim 5, wherein, in the scheme 1, the solvent is water or methanol;
and/or, in the scheme 1, the temperature of crystallization is 20 to 50°C, preferably 40°C or 50°C;
and/or, in the scheme 1, the mass volume ratio of the amorphous compound A to the solvent is 50 mg/mL;
and/or, in the scheme 2, the solvent is selected from the group consisting of methanol, tetrahydrofuran and DMSO;
and/or, in the scheme 3, the volume ratio of solvent to water is 3:1 to 1:1;
and/or, in the scheme 3, the volume ratio of DMSO to water is 1:1 to 1:4;
and/or, when the scheme 1 is adopted, the preparation method comprises the following steps: transforming a suspension of the mixture of the amorphous form of compound A and a solvent; and the solvent water or methanol; the temperature of stirring is 20 to 50°C, preferably 40°C; the mass volume ratio of the amorphous form of compound Ato the solvent is 50 mg/mL;
and/or, when the scheme 2 is adopted, the preparation method comprises the following steps: mixing the compound A with a solvent, and then slowly adding dropwise into an anti-solvent; the solvent is selected from the group consisting of methanol, tetrahydrofuran and DMSO; the anti-solvent is water; and the volume ratio of the solvent to water is 3:1 to 1:1;
and/or, when the scheme 3 is adopted, the preparation method comprises the following steps: adding the solution of compound A and a solvent into the aqueous solution A, and crystallizing, wherein the aqueous solution A is a suspension of the crystal seeds of crystalline form III of substance A and water; the solvent is DMSO; the volume ratio of DMSO to water is 1:1 to 1:4.

7. A crystalline form III of substance A, wherein, the crystalline form III of substance A is prepared according to the preparation method of claim 5 or 6.

8. The crystalline form III of substance A according to claim 7, wherein, the crystalline form III of substance A is the crystalline form III of the hydrate of compound A according to any one of claims 1 to 4.

9. A preparation method of the crystalline form V of the compound of formula A, wherein, the preparation is scheme A or scheme B;
scheme A comprises the following steps: transforming a suspension of amorphous form of the compound of formula A and a solvent into a crystalline form V of compound A at 20 to 50 °C; the solvent is water or nitrile solvent; the temperature of crystallization is preferably 50 °C;
scheme B comprises the following steps: volatilizing the solvent in the solution of the compound of formula A and the solvent into a crystalline form V of compound A; the solvent is alcohol solvent.

10. The preparation method according to claim 9, wherein,
in scheme A, the solvent is water or acetonitrile;
and/or, in scheme A, the mass volume ratio of the amorphous compound A to the solvent is 3.0mg/mL or 50mg/mL;
and/or, in scheme A, the temperature of crystal transformation is 50°C;
and/or, in scheme B, the solvent is methanol;
and/or, in scheme B, the temperature of crystal transformation is 50°C;
and/or, when the scheme A is adopted, the scheme comprises the following steps: transforming a suspension of the amorphous form of compound A and a solvent into the crystalline form V of the compound at 20 to 50 °C; the temperature of crystallization is 50° C, the solvent is water or acetonitrile; and the mass volume ratio of the amorphous form of compound A to the solvent is 50 mg/mL or 3.0 mg/mL;
and/or, when the scheme B is adopted, the scheme comprises the following steps: volatilizing the solvent in the solution of the compound A and the solvent into the crystalline form V of compound A; the solvent is methanol, the temperature is 50 °C.

11. A pharmaceutical composition comprising the crystalline form of the compound of formula A or the solvate thereof according to any one of claims 1 to 4 and/or the crystalline form III of substance A according to claim 7 or 8, and pharmaceutical excipient.

12. A use of the crystalline form of the compound of formula A or the solvate thereof according to any one of claims 1 to 4, the crystalline form III of substance A according to claim 7 or 8 in the manufacture of P2X3 receptor antagonist or a medicament, the medicament is a medicament can be used to protect, deal with, treat, or alleviate at least part of P2X3-mediated or activity related diseases of animals; alternatively, the medicament is a medicament can be used to treat pain, urinary tract disease, or respiratory disease.

13. The use according to claim 12, wherein the disease includes pain, urinary tract diseases and respiratory diseases; the pain preferably includes inflammatory pain, surgical pain, visceral pain, toothache, premenstrual pain, central pain, pain caused by burns, migraine or cluster headache; the urinary tract disease includes urinary incontinence, overactive bladder, dysuria and cystitis; the respiratory disease preferably includes respiratory disorder, including idiopathic pulmonary fibrosis, chronic obstructive pulmonary disease, asthma, bronchospasm and chronic cough.

14. The pharmaceutical composition according to claim 13, wherein, the side effects of taste disorders associated with treatment are reduced by administering the pharmaceutical composition.
